(12) **EUROPEAN PATENT SPECIFICATION**

(45) Date of publication and mention
of the grant of the patent:
**02.11.2006 Bulletin 2006/44**

(21) Application number: **02791492.8**

(22) Date of filing: **26.07.2002**

(51) Int Cl.:
*G01N 33/68* (2006.01)     *G01N 33/58* (2006.01)
*C12N 15/63* (2006.01)     *C12P 21/00* (2006.01)
*C12Q 1/68* (2006.01)

(86) International application number:
**PCT/EP2002/009424**

(87) International publication number:
**WO 2003/012451 (13.02.2003 Gazette 2003/07)**

(54) **PROTEIN ARRAYS, METHODS FOR THEIR PREPARATION AND METHODS FOR THE DETECTION OF INTERMOLECULAR INTERACTIONS**

PROTEIN-ARRAYS, VERFAHREN ZU IHRER HERSTELLUNG SOWIE VERFAHREN ZUM NACHWEIS INTERMOLEKULARER WECHSELWIRKUNGEN

RESEAUX DE PROTEINES, PROCEDES DE PREPARATION DE CES DERNIERS ET PROCEDES DE DETECTION D'INTERACTIONS INTERMOLECULAIRES

(84) Designated Contracting States:
**AT BE BG CH CY CZ DE DK EE ES FI FR GB GR IE IT LI LU MC NL PT SE SK TR**

(30) Priority: **27.07.2001 EP 01402050**

(43) Date of publication of application:
**28.04.2004 Bulletin 2004/18**

(73) Proprietor: **Université de Nantes
44035 Nantes (FR)**

(72) Inventors:
• **SAKANYAN, Vehary**
**F-44700 Orvault (FR)**
• **SNAPYAN, Marina**
**F-94400 Vitry-Sur-Seine (FR)**
• **GHOCHIKYAN, Anahit**
**Huntington Beach**
**CA 92647 (US)**
• **LECOCQ, Françoise-Michèle**
**F-44300 Nantes (FR)**
• **GUEVEL, Laetitia**
**F-44360 Saint-Etienne de Montluc (FR)**
• **WEIGEL, Pierre**
**F-44700 Orvault (FR)**
• **BRAUN, Frédérique**
**F-44000 Nantes (FR)**

(74) Representative: **Desaix, Anne et al**
**Ernest Gutmann - Yves Plasseraud S.A.S.**
**3, rue Auber**
**75009 Paris (FR)**

(56) References cited:
**WO-A-00/04382          WO-A-00/54046
WO-A-00/63413          WO-A-00/63418**

• **SILZEL J.W. ET AL: "Mass-sensing, multianalyte microarray immunoassay with imaging detection." CLINICAL CHEMISTRY, vol. 44, no. 9, 1998, pages 2036-2043, XP002196788**
• **ESSER M ET AL: "Infrared Fluorescent Labeling for use in Expression Analysis" ON-LINE POSTERS, WWW.LICOR.COM, [Online] 2001, XP002196789 Retrieved from the Internet: < URL:www.licor.com> [retrieved on 2002-04-17] cited in the application**
• **WADA TAKASHI ET AL: "Cloning of the RNA polymerase alpha subunit gene from Thermus thermophilus HB8 and characterization of the protein." JOURNAL OF BIOCHEMISTRY (TOKYO), vol. 125, no. 1, January 1999 (1999-01), pages 143-150, XP001073512 ISSN: 0021-924X**
• **LIPOWSKA M ET AL: "NEW NEAR-INFRARED CYANINE DYES FOR LABELLING OF PROTEINS" SYNTHETIC COMMUNICATIONS, MARCEL DEKKER, INC., BASEL, CH, vol. 23, no. 21, 1993, pages 3087-3094, XP002930077 ISSN: 0039-7911**

EP 1 412 758 B1

• SNAPYAN MARINA ET AL: "Dissecting DNA-protein and protein-protein interactions involved in bacterial transcriptional regulation by a sensitive protein array method combining a near-infrared fluorescence detection." PROTEOMICS, vol. 3, no. 5, 20 May 2003 (2003-05-20), pages 647-657, XP008020261 ISSN: 1615-9853

**Description**

The present invention relates to a method for the detection of intermolecular interactions between probe(s) and protein targets, said protein targets including at least one protein or polypeptide, which, when expressed *in vivo* into host cells, is toxic for said host cells or cannot be purified, or does not fold correctly. In particular, the invention results from the combination of the preparation of protein arrays with cell-free synthesized proteins and the detection of specific interactions using near-infra-red fluorescent dyes. The invention also relates to protein arrays and methods for their preparation.

The systematic sequencing of genomes has recently provided to the biologists, a high number of sequences of putative proteins of unknown functions. In parallel, by the use of functional genomics technologies, systematic screening for abnormal expression of sequences correlated to a specific pathology enabled to identify potential targets for new drugs, consisting of proteins which, for a part thereof, have unknown functions.

In order to develop and/or screen the potential drugs which could target these proteins whose abnormal expression is correlated to specific pathologies, there is a need in high-throughput methods for the detection of molecules, such as nucleic acids, proteins, small ligands and other compounds which bind to these protein targets.

Several methods have already been proposed to identify protein-DNA or protein-protein interactions both *in vivo* and *in vitro.* These methods are characterized in that they allow the analysis of a high number of interactions in parallel. Especially, improved versions of a two-hybrid system, originally based on a selective activation of reporter genes transcribed by RNA polymerase II, are widely used in proteomic researches (Vidal & Legrain, 1999; Joung *et al.,* 2000;). Also, phage-display technology, enabling the screening of short peptides interacting with other molecules, has been widely used for studying protein-protein and protein-DNA interactions.

More recently, protein array technology has become a powerful approach for the identification of high-throughput intermolecular interactions (Büssow *et al.,* 1998, Silzal *et al.,* 1998, MacBeath & Schreiber, 2000;Holt *et al.,* 2000; Mahlknecht *et al.,* 2001). Several methods for preparing protein arrays have been described. For example, one method is based on the binding of proteins on a nitrocellulose membrane at defined positions and the incubation of the membrane with $^{32}$P-labelled probe to identify protein-nucleic acid, protein-protein and protein-small ligand interactions (Ge, 2000, WO 0054046). After several washings, specific interactions between probes and targets are detected by autoradiography or by PhosphorImager.

In the above-mentioned methods, the proteins which are spotted as protein arrays are generally synthesized using *in vivo* cellular expression.

However, *in vivo* intracellular synthesis of proteins requires several steps, including insertion of the corresponding DNA into a vector molecule, its introduction into a given host, its expression and further purification of the expression product. Moreover, some genes may be toxic for host cells, or their expression product cannot be purified easily, for example, due to their hydrophobic structure, or do not fold correctly in such a way that they do not recover a natural conformation after *in vivo* synthesis and purification. This raises the question of providing methods for the preparation of protein arrays, which comprise a protein which cannot be synthesized *in vivo,* namely by intracellular expression. One solution is provided by *in vitro* chemical synthesis. However, the chemical synthesis is expensive and is not suitable for the synthesis of long polypeptides or proteins. This approach is thus limited for the synthesis of small peptides, generally up to 50 amino acids. Besides, this method does not provide possible maturation of proteins that normally occurs in cells.

Contrary to *in vivo* protein production, cell-free systems allow to synthesize proteins harmful for cell hosts (Zubay, 1973;). Cell-free synthesis has been used as an essential component for translation-based screening in polysome display, truncation test (Van Essen *et al.,* 1997), scanning saturation mutagenesis, site-specific incorporation of unnatural amino acids into proteins, stable-isotope labeling of proteins. Besides, cell-free systems have been used for identification of modified proteins by analysis of their electrophoretic migration (King *et al.,* 1997) or for identification of DNA-binding proteins by mobility shift assay (Lustig *et al.,* 1997). Both circular and linear exogenous DNAs can be used as templates for protein synthesis and a good protein yield can be reached from strong phage promoters in bacterial cell-free system. However, many factors affect the yield and the homogeneity of full-length synthesized proteins in a cell-free system and, therefore, its wider application is limited. Thus, considering the lower yield of protein synthesis using a cell-free protein synthesis system, *in vivo* cellular systems are generally preferred.

Indeed, the deposit of a high amount of stable proteins is considered as essential for the preparation of a protein array suitable to detect specific probe-protein interactions with a good sensitivity using the commonly used labeling methodology.

Till now, no protein array based on cell-free synthesized proteins has been described comprising serially produced proteins which are not producible *in vivo* either due to a toxicity for their host cells or for the difficulty to obtain a purified or correctly folded product.

Thus, there is still a need to develop new methodological approaches able to perform serial, rapid and sensitive detection of intermolecular interactions between probe(s) and protein targets, said targets including at least one protein or polypeptide which is not producible *in vivo* in host cells, and is toxic for said host cells or cannot be purified, or does not fold correctly.

The use of the term "serial detection" means that several distinct targets are tested in parallel for their interaction with at least one specific probe.

Preferably, more than ten targets are tested simultaneously.

Fluorescence methodology, as an alternative to radioactive and chemiluminescence's ones, is widely used for direct detection of labelled macromolecules and for screening of macromolecules targeted by dye-labelled probes. Recently, a detection method of nucleic acids and proteins by infrared fluorescence imaging has been developed by LI-COR, Inc. Reacting macromolecules labelled with near infrared (IR) fluorescent dyes with emission at approximately 700 nm and 800 nm can be detected by means of the LI-COR Odyssey IR Imager equipped with lasers that excite at 680 and 780 nm. The infrared region has a lower background, as compared to the lower wave-length dyes used in other fluorescent detection methods, thus providing a higher signal-to-noise ratio and allowing to detect target molecules with a higher sensitivity. Validity of the technology has been shown for the detection of nucleic acids and proteins bound to membranes as well as for the detection of protein targets with IRD-labelled antibodies (Esser *et al.,* 2000; Schutz *et al.,* 2000).

The invention results from the finding that *in vitro* synthesized proteins can be used for the preparation of protein array. Indeed, the inventors have shown that use of arrays based on *in vitro* synthesized proteins, in combination with the IR dyes, provide a highly sensitive and rapid method for detection of intermolecular interactions between probes and protein targets.

Especially, the invention provides a method for the preparation of a protein array comprising protein targets, preferably including one protein or polypeptide unproducible *in vivo* which, when expressed *in vivo* into host cells, is toxic for said host cells or cannot be purified, or does not fold correctly, said method comprising:

a) synthesizing in parallel protein targets using a cell-free protein synthesis method with an excess of the $\alpha$ subunit of RNA-polymerase according to other subunits of the like;

b) recovering the synthesized proteins or polypeptides; and,

c) preparing a protein array by binding synthesized proteins or polypeptides to a support at defined positions.

As used herein, the term "protein array" refers to a support comprising a set of proteins or polypeptides bound at defined positions, each position corresponding to one specific sequence of polypeptide or protein. One or more positions can correspond to the same sequence of polypeptide or protein. A protein array can comprise up to thousands different polypeptides or proteins.

According to the invention, the protein targets refer, interchangeably, to the proteins or polypeptides which are bound to the support.

The one skilled in the art can choose any protein or polypeptide, for which intermolecular interaction with specific probe(s) is questioned. These targets can be for example all the proteins encoded by the genome of an organism. These targets can also be a specific family of proteins, according to their function in the cell, such as protein kinases, transcription factors, receptors and the like. These targets can also be proteins involved in diseases, and especially proteins from pathogenic organisms or proteins with abnormal expression in cancer, cardio-vascular, neuro-degenerative or genetic pathologie. These targets can also be proteins exhibiting various catalytic activities and/or enabling the binding of small ligands or cofactors or short peptides.

Preferably, the protein targets include proteins or polypeptides which are not obtainable using an *in vivo* cellular expression system and which have at least an amino acid sequence longer than 30 amino acids, preferably longer than 50 amino acids.

The protein targets comprise at least one protein target which, when expressed *in vivo* into host cells, is toxic for said host cells or cannot be purified, or does not fold correctly.

As used herein, the term "cannot be purified", means that conventional purification procedures cannot provide a purified protein containing less than 10% of impurities, or cannot be purified without affecting its correct folding such as membrane proteins.

According to the invention, a protein which does not fold correctly is a recombinant protein which has not substantially the same binding properties as a corresponding native protein purified from homologous host-organism.

More particularly, proteins or polypeptides that are toxic or cannot be purified or do not fold correctly comprise mis-folded wild-type or mutant oligomeric or monomeric proteins, belonging to regulatory, membrane-attached, secretory and other proteins, because of their expression *in vivo,* in particular, in heterologous cytoplasmic environment. In particular, *in vivo* multiplication of a given gene under selective pressure, said given gene encoding a protein harmful for the host cell, may generate mutations in the gene and the corresponding mutated protein which do not reflect real binding properties of the wild-type protein.

Thus, according to the method of the invention, the target proteins are synthesized *in vitro* using a cell free protein synthesis method, allowing rapid parallel synthesis of number of proteins, including those which, when expressed *in vivo* into host cells, are toxic for said host cells or cannot be purified, or do not fold correctly.

As used herein, a cell-free protein synthesis method refers to any method for cell-free synthesis of a desired protein from a ribonucleic acid (RNA) template or a deoxyribonucleic acid (DNA) template. Hence, it can refer either to *in vitro* transcription-translation or *in vitro* translation methods. These methods are opposed to *in vivo* protein synthesis methods involving the culture of recombinant cells expressing the desired proteins, and to chemical synthesis methods. Examples of eukaryotic *in vitro* translation methods are the methods involving the rabbit reticulocytes (Pelham and Jackson, 1976) or wheat germ lysate (Roberts and Paterson, 1973). The *E. coli* S30 extract method described by Zubay, (1973) is an example of a widely used prokaryotic *in vitro* transcription-translation method.

Lesley *et al.,* (1991) optimised the Zubay (1973) *E. coli* extract for use with PCR fragments and other linear DNA templates by preparing a bacterial extract from a nuclease-deficient strain of *E. coli.* Also, improvement of the method has been described by Kigawa *et al.,* (1999).

In a preferred embodiment of the invention, said cell-free protein synthesis method comprises the use of a cell-free extract, preferentially a bacterial cell-free extract, more preferably *E. coli* cell-free extract. The term cell-free extract as used herein defines any preparation comprising the components of protein synthesis machinery wherein such components are sufficient to enable translation of a messenger ribonucleic acid encoding a desired protein. Optionally, the cell-free extract comprises components which further allow transcription of the DNA encoding the desired protein.

When the template is a DNA, it comprises at least the sequence of an Open Reading Frame (ORF) encoding the desired protein and the sequences appropriate for transcription and translation initiation. Optionally, the DNA template may comprise short sequences encoding tags for facilitating further purification of the synthesized protein.

In a preferred embodiment, the DNA template also comprises a strong bacterial promoter with at least one UP element, preferentially the promoter of the *argCo* gene from *Bacillus stearothermophilus.*

The linear DNA template can also be a PCR-produced DNA template comprising the ORF encoding said protein or polypeptide, and an additional sequence which is in a preferred embodiment, at least 3 bp, preferably longer than 100 bp and more preferably longer than 200 bp. This elongation of PCR-produced DNA template has been shown to improve the yield and the homogeneity of *in vitro* protein synthesis using a bacterial cell free extract.

Each protein target can thus be synthesized in parallel by, first, amplifying the gene encoding the desired protein or polypeptide and second, carrying out cell-free protein synthesis using the amplification product as a DNA template.

It is known in the art that adding purified RNA polymerase holoenzyme may improve the yield of protein synthesis. For example, purified T7 RNA polymerase can be added to the reaction mixture when carrying out cell-free protein synthesis using a T7 phage promoter. However, it has also been shown that adding purified thermostable RNA polymerase, preferably *T. thermophilus,* enables similar yield, when corresponding promoter sequences are applied.

Thus, in a preferred embodiment of the method, wherein a bacterial cell-free extract is used, a purified thermostable RNA polymerase, preferably from *T. thermophilus,* is added into the bacterial cell-free extract.

It has also been shown that, surprisingly, adding specifically purified α subunit of RNA polymerase in the cell-free extract in excess to the host RNA polymerase holoenzyme of the like provides better yield of protein synthesis, than with T7 RNA polymerase holoenzyme, when corresponding promoter sequences are applied.

In one preferred embodiment wherein a bacterial cell-free extract is used, the method of the invention is characterized in that purified α subunit of RNA polymerase is added in the cell-free extract in excess to other subunits of the like.

Alternatively, in another embodiment wherein a bacterial cell-free extract is used, the method of the invention is characterized in that cell-free extract is prepared from host cells overexpressing a gene encoding α subunit of RNA polymerase.

The protein targets are then recovered after cell-free synthesis. They can be purified prior to the deposition on the support or they can be partially purified or directly deposited without any purification. In particular, they can be purified using column affinity chromatography methods.

In order to form a protein array, the protein targets are bound to a support at defined positions. Depending on the type of the support, the target might be either tightly bound to the support or needs to be chemically linked to the support. An example of a support requiring no chemical linking is an organic membrane, preferably a nitrocellulose membrane. In other examples, a linking or binding method must be performed to ensure the binding of the polypeptides. Examples of linking methods are described in the prior Art, as well as the nature of supports appropriate for the binding of the protein targets.

As an example for the preparation of a protein macroarray, protein targets ranging from 0.1 pmole to 400 pmole can be deposited on nitrocellulose membrane by vacuum without using any specific linker. The protein microarray can also be prepared using a spotter, such as the arrayer Affymetrix GMS417.

One advantage of the vacuum blotting of cell-free synthesized proteins as compared with spotting approaches is that both addition and binding proteins to the support can be done on a relatively large surface, easily controlled even for diluted samples, allowing the binding of equimolar quantities of cell-free synthesized proteins to the support.

One advantage of the method of the invention is that protein targets can be directly deposited after their synthesis without purification, as crude extracts obtained from in vitro expression systems.

Accordingly, a protein array is provided, comprising at least one non-purified protein or polypeptide not obtainable by *in vivo* synthesis method but obtainable with a cell-free protein synthesis method.

A protein array can be obtained, in which protein or polypeptide bound to the support, when expressed *in vivo* into host cells, is toxic for said host cells or cannot be purified, or does not fold correctly.

In a specific embodiment the protein array contains a protein or polypeptide which is not obtainable using an *in vivo* cellular expression system, has an amino acid sequence longer than 30 amino acids, preferably longer than 50 amino acids.

In another specific embodiment, a protein array is obtainable by the method for the preparation of a protein array as defined above.

Indeed, the present invention relates to a simple, rapid and sensitive method for the serial detection of intermolecular interactions between protein or polypeptide targets and probes.

The present invention encompasses also a method for the detection of intermolecular interactions between probe(s) and protein targets, said protein targets, preferably including at least one protein or polypeptide unproducible *in vivo* which, when expressed *in vivo* in host cells, is toxic for said host cells or cannot be purified, or does not fold correctly, said method combining the steps of

a) providing protein targets by serially *in vitro* synthesizing them, using a cell-free protein synthesis method with an excess of the α subunit of RNA-polymerase compared to other subunits of the like;

b) binding to a support at defined positions, said protein targets to prepare a protein array ;

c) providing probe(s) labelled with a near-infrared fluorescent dye,

d) contacting said protein array with the labelled probe(s) in a medium suitable to allow a specific binding, and

e) detecting on the protein array, the protein target(s) to which the labelled probe(s) specifically binds using an Infrared imager device.

Thus, the method allows to detect any intermolecular interactions between the protein targets and the probe. As used herein, "intermolecular interactions" or "specific binding" between a probe and a protein target refer to a physical interaction between two molecules which cannot be dissociated using usual washing conditions comprising incubation in a low stringency buffer, for example buffer A100 as described in Ge, 2000, at least 1 hour at room temperature.

According to the method of the invention, the protein targets are synthesized using cell-free protein synthesis systems, inducing especially bacterial cell-free extract, preferably *E. coli* cell-free extract, as described above. Similarly, the preparation of the protein array in step (b) is carried out as described above.

The term "probe" refers to a molecule which might bind with one or more polypeptides or proteins of the array in order to allow the detection of bound or reacting complexes. This probe can be any type of molecule. According to a preferred embodiment of the invention, said probe molecule is a nucleic acid, a polypeptide or a protein, bound directly or indirectly with a near-infrared fluorescent dye. Preferentially, a probe molecule is bound directly or indirectly with near infrared fluorescent dyes with emission at approximately 700 nm and 800 nm. These dyes can be detected, by means for example, of the Li-COR Odyssey Infrared Imager equipped with diode lasers that excite at 680 and 780 nm. Optionally, different probes labelled with different dyes can be pooled simultaneously to detect intermolecular interactions between the targets and said different probes.

Preferably, near infrared fluorescent dyes have wave length superior than 680 nm, preferably in the range between 680 nm and 800 nm.

The protein array is incubated in an incubation buffer comprising the probe(s), said incubation buffer consisting of a medium suitable to allow specific binding. After a period of time for incubation which can vary depending on the amount of targets and probes, the protein array is washed to avoid non specific interactions and very weak interactions.

According to the invention, the binding is considered to be specific when said binding is observed in an incubation buffer containing a non-specific competitor, *i.e.,* a molecule which binds weakly to any protein or polypeptide. An example of an incubation buffer for the detection of protein-DNA interactions is the following: 20 mM Tris-HCl pH 7.9, 50 mM NaCl, 50 mM KCl, 0.1 mM DTT, 0.005% surfactant P20 (Biacore) and 25 μg/ml of a sonicated salmon DNA used as competitor DNA. An example of an incubation buffer for the detection of protein-protein interactions is the PBS containing 1% BSA and 0.1% Tween 20 (LI-COR, INC.) or the A100 buffer as described by Ge (2000) containing 1% non-fat milk.

The probes labelled with infrared fluorescent dyes remain bound to the proteins to which they interact specifically and generate, on the protein array a signal which can be detected using a device allowing infrared detection. As an example, a device allowing infrared detection is the IRD Imager developed by LI-COR ®.

Cell-free protein synthesis enables to provide normalized amounts of protein target before binding to the support.

Thus, in another specific embodiment, the method of the invention is characterized in that it further comprises steps for normalization of the amount of synthesized protein prior to binding to a support, said normalization steps consisting of:

a) labelling each synthesized protein, preferably by supplying a radioactive amino acid in the cell-free system,
b) quantifying each labelled protein, and
c) providing equivalent amount of each synthesized protein for normalization prior to binding to a support.

Another advantage of the method of the invention is that multiple probes can be used simultaneously, the probes being labelled with different specific IRDyes of different wave-length. Accordingly, in one specific embodiment of the method of the invention, at least two different probes are used, said probes being labelled with IRDyes of different wave-lengths.

The present invention also relates to the use of *in vitro* synthesized proteins or polypeptides for the preparation of protein arrays wherein, at least one of the proteinsor polypeptides when expressed *in vivo* in host cells, is toxic for said host cells or cannot be purified, or does not fold correctly.

The proteins are synthesized *in vitro* as defined above for their use in the preparation of protein arrays.

Especially, proteins or polypeptides for use in the preparation of protein arrays as above-mentioned, are synthesized from a PCR-produced DNA template, said PCR produced DNA template comprising an ORF encoding said protein or polypeptide, and an additional sequence which is at least 3. bp long, preferably longer than 100 bp, and more preferably longer than 200 bp, located immediately downstream the stop codon of the gene encoding said protein or polypeptide.

A purified $\alpha$ subunit of RNA polymerase is added in the cell-free extract in excess to the RNA polymerase holoenzyme for the preparation of the proteins or polypeptides for use in the protein arrays.

The method can be used to screen DNA fragments binding to the $\alpha$ subunit of bacterial RNA polymerase. The $\alpha$ subunit of bacterial RNA polymerase is synthesized *in vivo* in host cell expression systems or *in vitro* in a coupled transcription-translation system and is immobilized with or without purification on support to provide a protein array useful for the screening of strong promoters, said strong promoters being **characterized in that** they binds specifically with $\alpha$ subunit, said $\alpha$ subunit being not assembled with other subunits of the RNA polymerase.

The screening of DNA fragments binding to $\alpha$ subunit is more specifically useful to identify UP elements, or consensus promoter sites -10 and -35 sites and the AT rich sequences located upstream the -35 site of strong promoters.

The method can be used for the detection of DNA fragments binding the cAMP receptor protein (CRP). CRP is synthesized *in vivo* in host cell expression systems or *in vitro* in a coupled transcription-translation system and is immobilized with or without purification on support to provide a protein array useful for the screening of strong promoters, said strong promoters being characterized in that they bind specifically with CRP.

The examples below illustrate some preferred embodiments of the invention without limiting the scope of the invention. Especially, the results presented in the examples show that the method of the invention is particularly sensitive and can detect weak intermolecular interactions, for example with $K_D$ less than $10^{-7}$M for DNA-protein interactions and protein-protein interactions.

## LEGENDS OF THE FIGURES

**Figure 1:** A drawing showing the transcriptional and translational signals identified for the *B. stearothermophilus argC* gene.

An UP DNA element is shown in *italic.* -35 and -10 promoter sites as well as a Shine-Dalgamo site are underlined. Position of the oligonucleotide primers used for the PCR amplification of the 89-bp, 59-bp and 39-bp promoters are shown by arrow. *argCo* operator sequence is shown in thick line.

**Figure 2:** An image of a protein array prepared with cell-free synthesized $^{35}$S-labelled proteins immobilized on a nitrocellulose membrane.

*T. maritima* GntRTm0439 (lines 1 - 6) and *T. maritima* LacITm1856 (7 - 12) cell-free synthesized His-tagged proteins were purified by affinity chromatography, diluted consecutively 2-fold (from A to H), blotted by vacuum on a nitrocellulose membrane and cut out into strips. The strips were directly used for autoradiography (lines 1 and 7) or consecutively rinsed in binding buffer (lines 2 and 8) and washed once or twice with A100 (lines 3 and 9 or 4 and 10), A500 (lines 5 and 11) and A1000 (lines 6 and 12). Autoradiography was performed with BioMax MS film for 30 min.

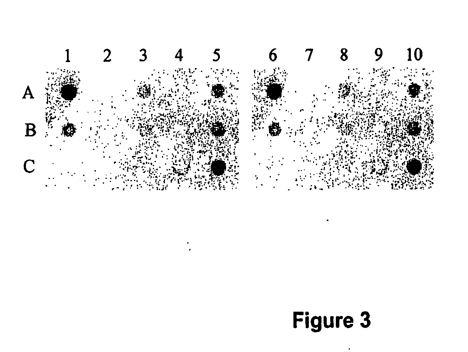

**Figure 3:** An image of immobilized protein showing ArgR protein interaction with a biotin-labelled *T. neapolitana*

*argRo* operator DNA.

His-tagged ArgR proteins of *B. stearothermophilus* (lines 1 and 6) or *T. neapolitana* (lines 3 and 8) were purified from recombinant *E. coli* BL21 (DE3) cells. A His-tagged ArgR of *T. neapolitana* was cell-free synthesized and purified by affinity-chromatography (slots 5A and 10A). Slots 5B and 10B - purified S30 extracts without synthesized ArgR, and slots 5C and 10C - non-purified S30 extracts without cell-free synthesized ArgR. Slots 1A, 3A, 6A and 8A - contain 100 pmol protein; slots 1B, 3B, 6B and 8B - contain 20 pmol protein; slots 1C, 3C, 6C and 8C - contain 2 pmol protein. A biotin-labelled *T. neapolitana argRo* operator DNA (final concentration 10 ng/ml) was incubated with membranes at 4°C (lines 1 - 5) or 60°C (lines 6 - 10) overnight.

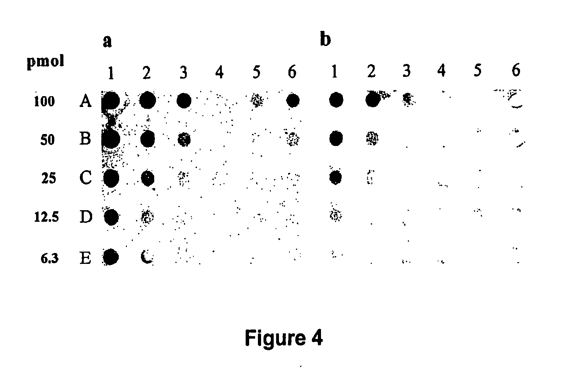

**Figure 4:** An image of a protein array showing DNA-protein interactions with IRD-800 labelled 59-bp DNA (**a**) or a 39-bp DNA (**b**) of *B. stearothermophilus argCo.*

1 - ArgR of *T. neapolitana;* 2 - ArgR of *B. stearothermophilus*; 3 - a chimeric ArgR-EbE; 4 - GroES of *T. neapolitana;* 5 - $\alpha$ subunit of *E. coli* RNA polymerase; 6 - CRP of *E. coli.* The serially 2-fold diluted protein samples were blotted on the nitrocellulose membrane, the membrane was divided into two halves and probed with respect to one of the two IRD-labelled probes.

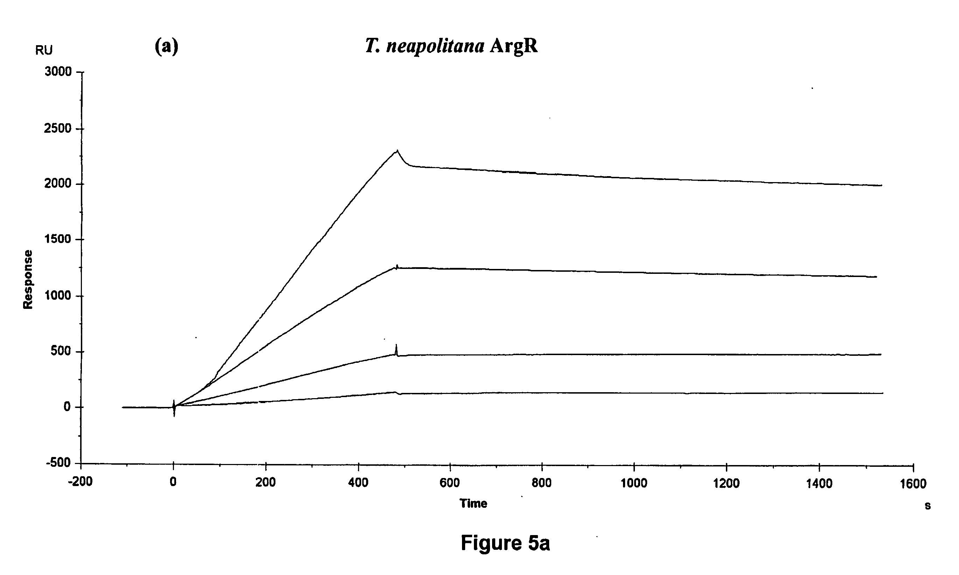

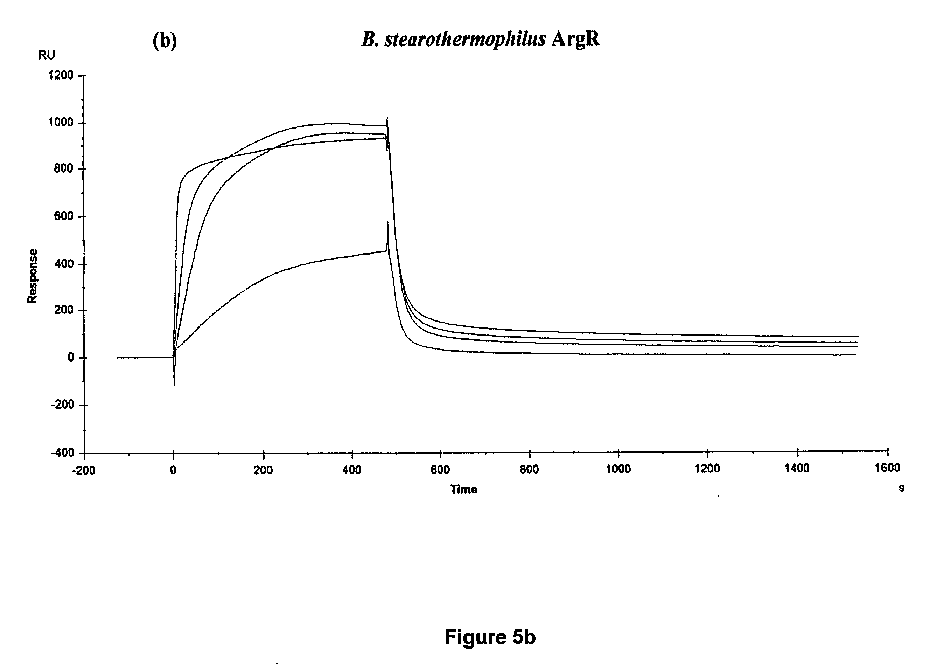

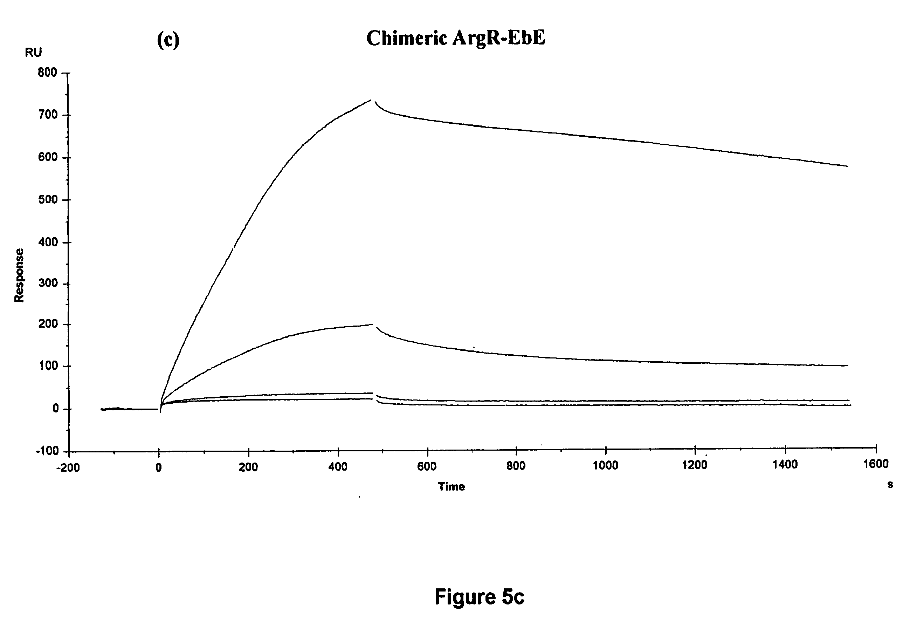

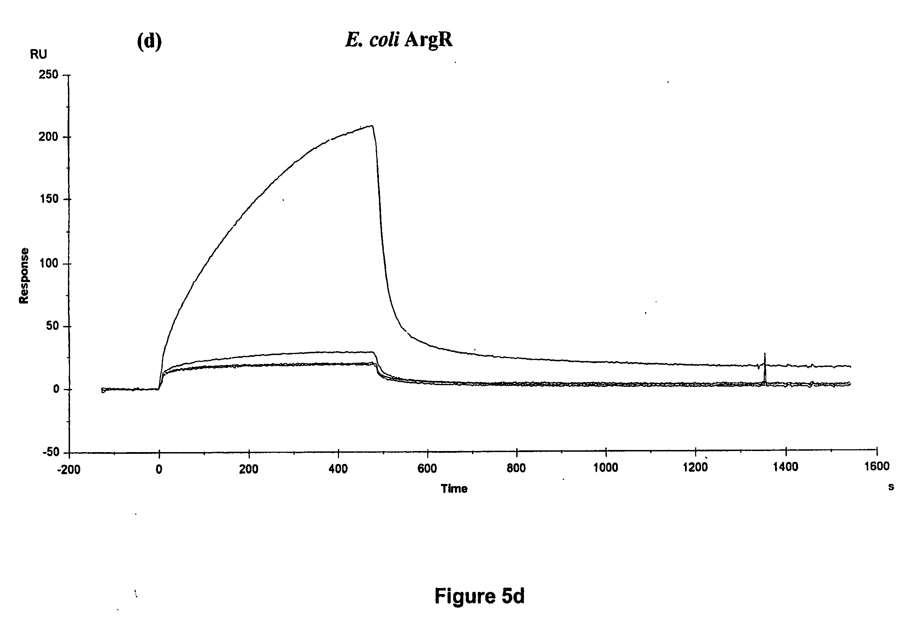

**Figure 5:** Sensorgrams showing binding of wild-type ArgR proteins of *T. neapolitana, B. stearothermophilus, E. coli* and a chimeric ArgR-EbE with respect to the *B. stearothermophilus argCo* operator DNA.

Purified His-tagged proteins were injected at 10 mM, 25 mM, 50 mM and 100 mM in presence of 5 mM L-arginine.

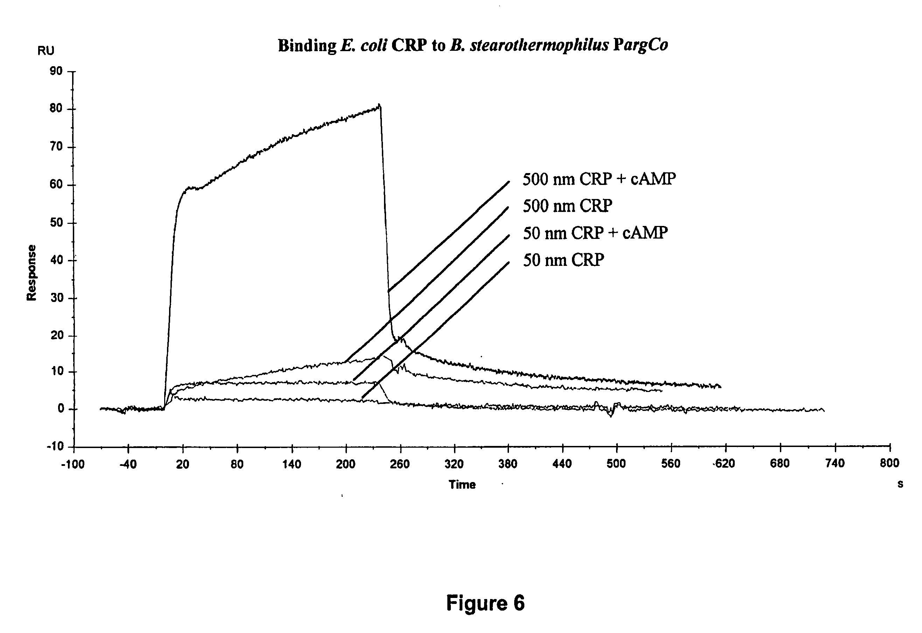

**Figure 6:** Sensorgram showing *E. coli* CRP interaction with respect to the *B. stearothermophilus argCo* operator DNA.

Purified His-tagged CRP was injected in absence or presence of 5 mM cAMP.

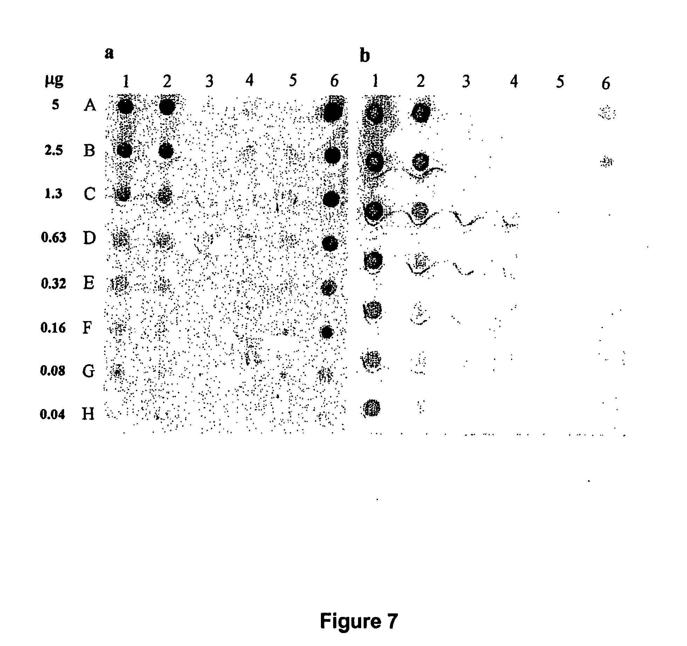

**Figure 7:** An image of a protein array showing subsequently protein-protein and DNA-protein interactions.

Proteins were serially diluted and blotted to the nitrocellulose membrane: 1 - ArgR of *T. neapolitana;* 2 - ArgR of *B. stearothermophilus*; 3 - ArgR of *E. coli*; 4 - GroES of *T. neapolitana;* 5 - $\alpha$ subunit of *E. coli* RNA polymerase; 6 - CRP of *E. coli.* The membrane was first used to probe with Cy5.5-labelled $\alpha$ subunit of *E. coli* RNA polymerase (**a**), then washed and reprobed with a IRD-800-labelled 39-bp DNA carrying a shortened *argCo* operator (**b**). 5 $\mu$g of deposited His-tagged proteins in the slots A correspond to 290 pmol of ArgR proteins, 500 pmol of GroES, 140 pmol of $\alpha$ subunit or 208 pmol of CRP.

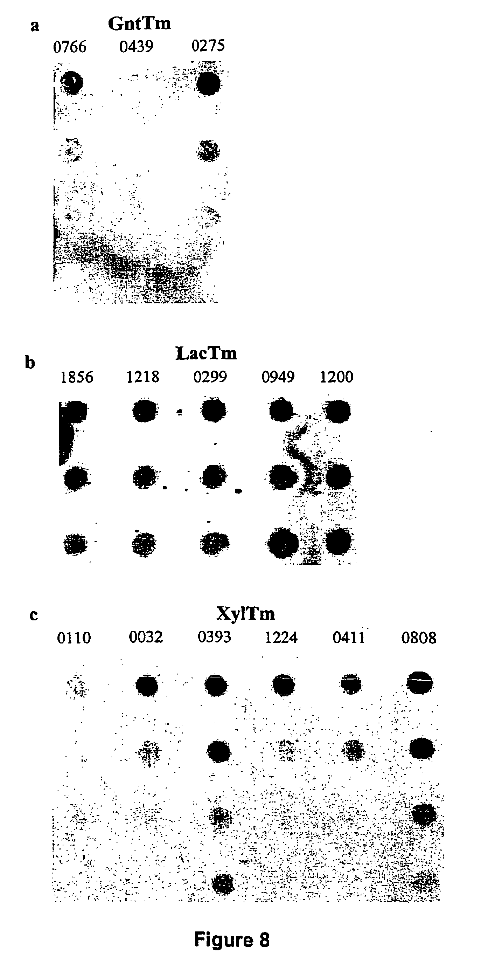

**Figure 8:** An image of cell-free synthesized protein array showing DNA-protein interactions.

Equimolar quantities (for monomer) of cell-free synthesized *T. maritima* putative proteins belonging to Gnt (**a**), Lac (**b**) and Xyl (**c**) family of regulators were serially 2-fold diluted and blotted on a nitrocellulose membrane. Incubation was carried out with respect to IRD-800 labelled operator DNA probes of *E. coli gntKo, lacIo* and *xylFo,* respectively at 60°C, overnight.

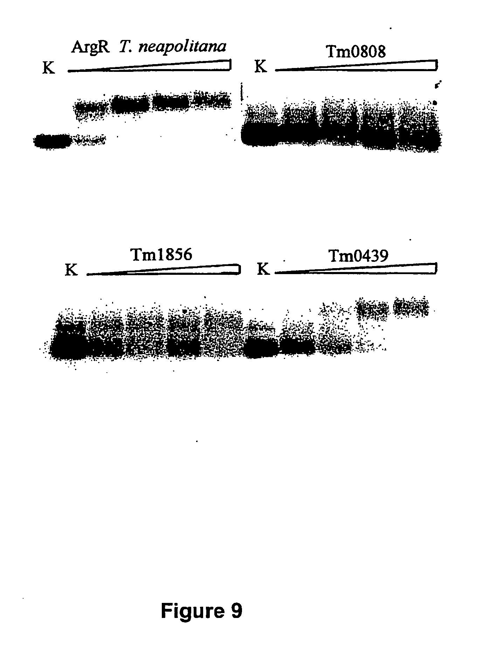

**Figure 9:** Non-denaturant agarose gel showing interaction of cell-free synthesized proteins with respect to operator DNAs.

His-tagged purified proteins were synthesized in a cell-free system and probed with respect to Dig-labelled operator DNA probes in DNA-binding buffer at 37°C for 30 min. ArgR binding was performed in presence of 10 mM L-arginine in binding and running buffers.

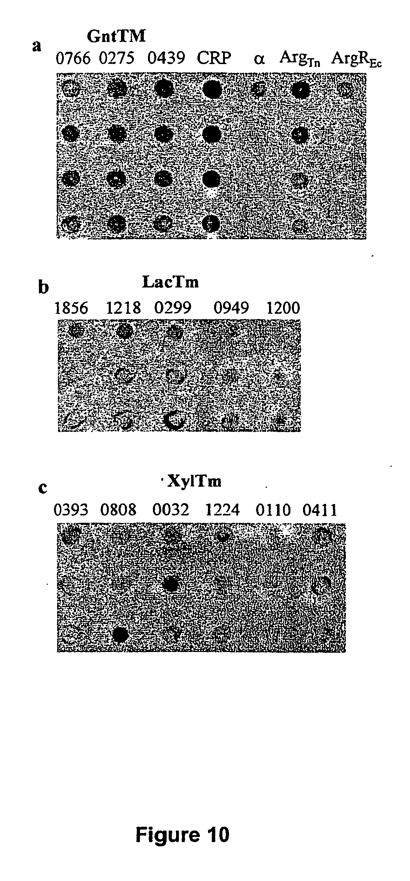

**Figure 10:** An image of cell-free synthesized protein array showing protein-protein interactions.

Equimolar quantities of cell-free synthesized *T. maritima* putative proteins belonging to Gnt (**a**), Lac (**b**) and Xyl (**c**) family of regulators were serially diluted and blotted on a nitrocellulose membrane. Incubation was performed with the Cy5.5-labelled $\alpha$ subunit of *E. coli* RNA polymerase at room temperature for 1 h. Non-diluted samples of CRP and $\alpha$ subunit of *E. coli* RNA purified from cells contain 200 pmol of proteins.

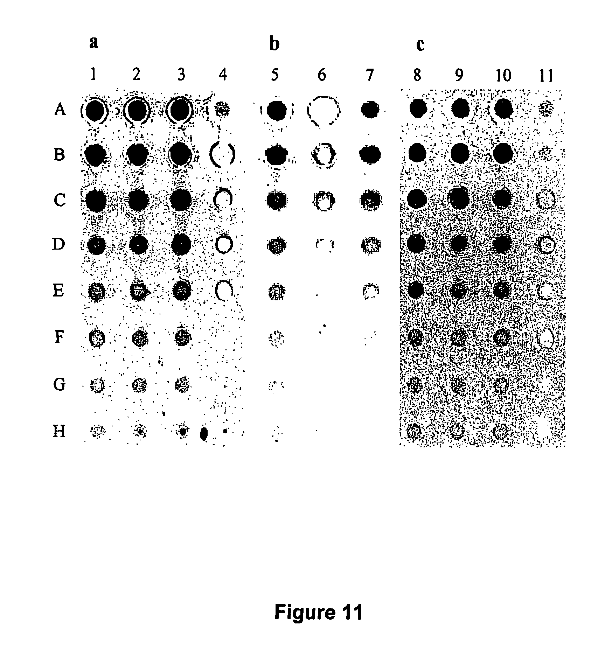

**Figure 11:** An image of a protein array showing ligand-protein interactions in rat cell-membrane homogenates using IRD-800 labelled rat secondary antibodies.

The immobilized homogenates were first bound to HNK-1 carbohydrate (**a**) or to 8 amino acid mimic protein (**c**), then these membranes were incubated with corresponding anti-HNK-1 or anti-mimic primary rat antibodies and then probed with IRD-800 labelled secondary antibodies. The membrane (**b**) was incubated with anti-GABAR primary antibodies and then probed with IRD-800 labelled secondary antibodies.

1, 5 and 8 - homogenates with concomitant expressed GABA$_B$R1a and GABA$_B$R2a receptors;

2, 7 and 9 - homogenates with concomitant expressed GABA$_B$R1b and GABA$_B$R2a receptors;

3 and 10 - laminin (the initial amount is 100 $\mu$g);

4, 6 and 11 - homogenates without expressed GABA receptors.

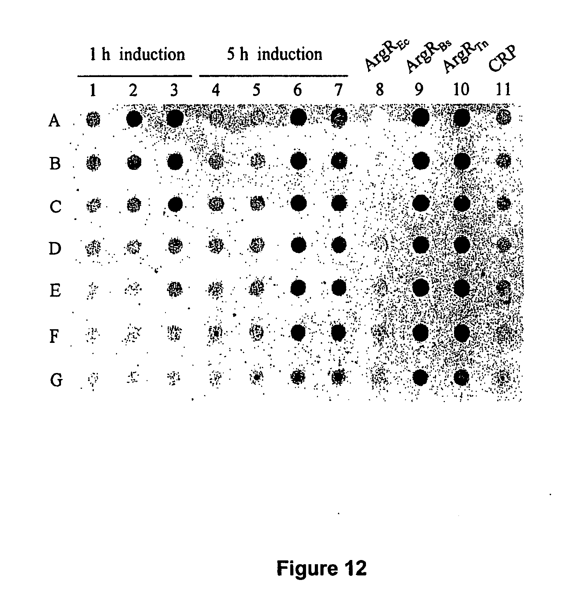

**Figure 12:** An image of a protein array showing the detection of DNA-protein interactions using extracts of *E. coli* BL21 (DE3) cells.

Concentration of a total or purified proteins added in A slots was 63 $\mu$g, except A2 in which 41 $\mu$g total protein was added because of a slower growth of *E. coli* (pET-ArgR$_{Bs}$-His). Incubation was carried out with a IRD-800 labelled 39-bp DNA of *B. stearothermophilus argCo* at room temperature overnight.

Vertical lines 1, 2 and 3, respectively 1 h IPTG-induced samples of *E. coli* (pET-ArgR$_{Ec}$-HIS), *E. coli* (pET-ArgR$_{Bs}$-His) and *E. coli* (pET-ArgR$_{Tn}$-HIS); 4, 5, 6 and 7, respectively 5 h IPTG-induced samples of *E. coli*, *E. coli* (pET-ArgR$_{Ec}$-HIS), *E. coli* (pET-ArgR$_{Bs}$-His) and *E. coli* (pET-ArgR$_{Tn}$-HIS); 8, 9 and 10- respectively, pure His-tagged ArgR of *E. coli*, *B. stearothermophilus* and *T. neapolitana*; 11 - pure His-tagged *E. coli* CRP.

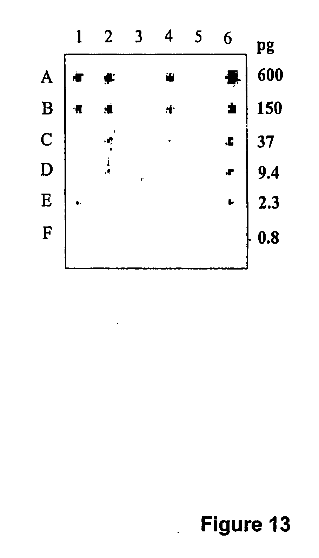

**Figure 13:** An image of a glass-slide microarray showing near-infrared detection of protein-protein interactions.
Spots 1, 2, 3, 4, 5 and 6 - respectively, correspond to *T. neapolitana* ArgR synthesized in a cell-free system or to, *B. stearothermophilus* ArgR, *E. coli* ArgR, *T. neapolitana* GroES, *E. coli* α subunit of RNA polymerase and CRP synthesized in *E. coli* cells. The amount of deposited proteins synthesized *in vivo* is shown in pg. Samples were diluted 4-fold and binding was carried out with Cy5.5-labelled *T. neapolitana* GroEL at room temperature for 1 h.

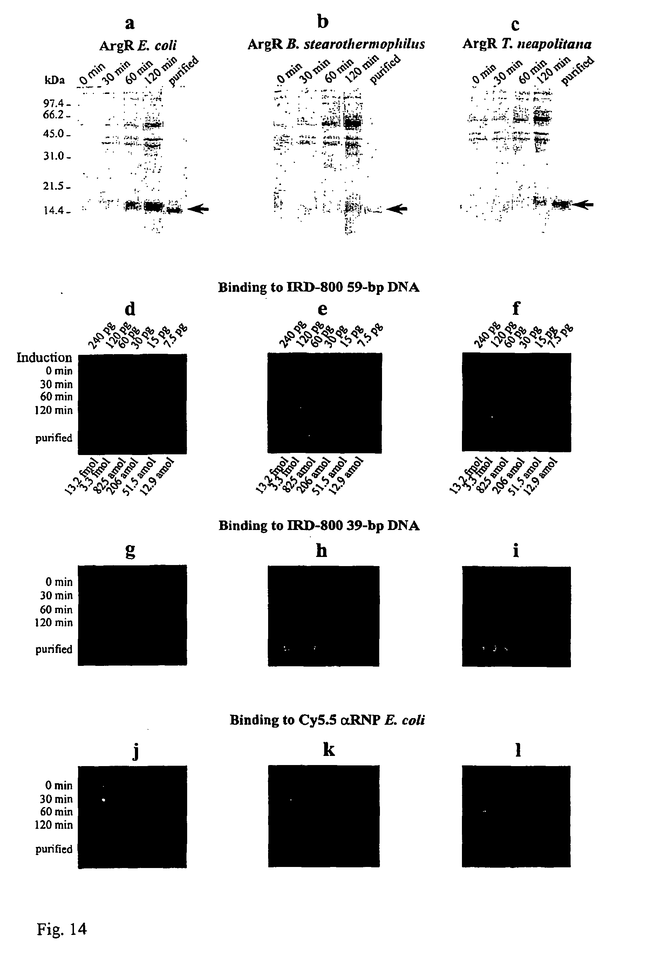

**Figure 14:** SDS-PAGE analysis and fluorescence detection of DNA-protein and protein-protein interactions on arrays with spotted ArgR repressors.
80 μg of a total protein (crude extracts) of non-induced and 30 min, 60 min and 120 min IPTG-induced samples of the *E. coli* BL21 (DE3) strain carrying the *argR* gene of *E. coli* (**a**), *B. stearothermophilus* (**b**) or *T. neapolitana* (**c**) were loaded on a polyacrylamid gel; the last lane contains 5 μg of a purified His-tagged protein; molecular mass references are indicated in kDa. Protein chips were prepared with the same crude extracts by a serial 2-fold dilution or with pure proteins by a serial 4-fold dilution. A total protein in spotted cell extracts is shown in pg, the amount of spotted pure proteins is shown in fmol and amol. Binding reactions were carried out with a 59-bp IRD-800 labelled DNA carrying the entire *B. stearothermophilus PargCo* region (**d, e** and **f**), or with **a** 39-bp IRD-800 labelled DNA carrying a shortened P*arg*Co region (**g, h** and **i**) or a Cy5.5 labelled *E. coli* RNA polymerase α subunit (**j, k** and **l**) in the presence of 10 mM arginine.

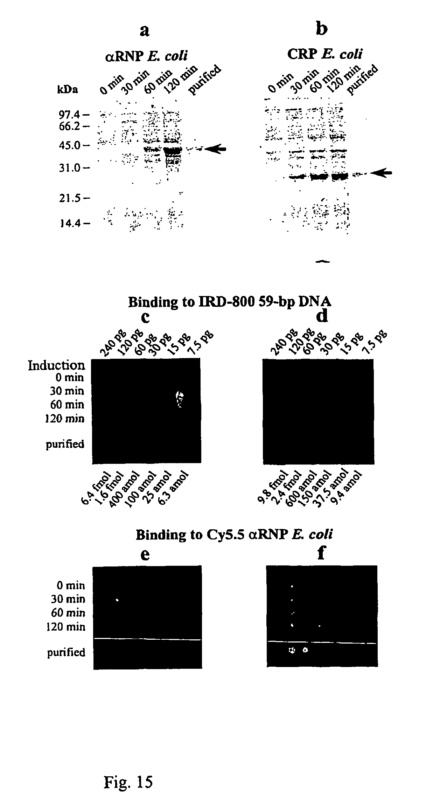

**Figure 15:** SDS-PAGE analysis and fluorescence detection of DNA-protein and protein-protein interactions on arrays with spotted *E. coli* RNA polymerase α subunit and CRP.
Binding reactions were carried out with a 59-bp IRD-800 labelled DNA carrying the *B. stearothermophilus PargCo* promoter-operator region (**c** and **d**) or with a Cy5.5 labelled *E. coli* RNA polymerase α subunit (**e** and **f**). Binding CRP chips was performed in the presence of 5 mM cAMP. Other descriptions are as in Figure 14.

## EXAMPLES

### A. Materials and Methods

#### A.1 Bacterial strains, plasmids and growth conditions.

Bacterial strains and plasmids are described in Table 1.

Table 1. Bacterial strains and plasmids used in this study.

| Strain/plasmid | Relevant genotype/description | Reference/source |
|---|---|---|
| *E. coli* K12 XA4 | F⁻ argA *nalA*λ⁻λ⁸ *trpR hsdR* | Laboratory collection |
| *E. coli* K12 Top10 | *F' mcrA Δ(mrr-hsdRMS-mcrBC) φ80lacZΔM15 ΔlacX74 deoR* | Invitrogen |
| | *recA1 araD139 Δ(ara-leu)7697 galU galK rpsL endA1 nupG* | |
| *E. coli* BL21 (DE3) | F *ompT hsdS$_B$* (r$_B$⁻ m$_B$⁻) gal (λclts857 *ind1* Sam7 *nin5 lac* UV5-T7 gene 1) | Novagen |
| *E. coli* BL21Z | F⁻ *ompT hsdS$_B$* (r$_B$⁻ m$_B$⁻) *gal dem Δ(lac-pro)::Tn10* | Laboratory collection |
| *E. coli* BL21 Star RecBCD (DE3) (pRIL) | F⁻ *ompT hsdS$_B$* (r$_B$⁻ m$_B$⁻) *gal dcm me131* (DE3) *recBCD* | Laboratory collection |
| *T. neapolitana* DSM5068 | Wild type | Laboratory collection |
| *T. maritima* MSB8 | Wild type | Laboratory collection |
| *B. stearothermophilus* NCIB8224 | Wild type | Laboratory collection |
| pCR4-TOPO | *bla kan,* PCR cloning vector | Invitrogen |
| pHAV2 | pBTac2 vector, carrying the *B. stearothermophilus argC* gene | Savchenko *et al.*, |
| | downstream P*argC* promoter | 1998 |
| pET19b | bla, T7 promoter expression vector | Novagen |
| pET21d(+) | *bla,* T7 promoter expression vector | Novagen |
| pET21d-ArgR$_{Tn}$-His | *bla,* T7 promoter expression vector, carrying the | Dimova *et al.*, 2000 |

(continued)

| Strain/plasmid | Relevant genotype/description | Reference/source |
|---|---|---|
| pET21d-ArgR$_{8s}$-His | *T. neapolitana argR* gene in fusion with C-terminal His-tag <br> *bla,* T7 promoter expression vector, carrying the *B. stearothermophilus argR* gene in fusion with C-terminal <br> His-tag | Karaivanova *et al.,* 1999 |
| pET21d-ArgR$_{Ec}$-His | *bla,* T7 promoter expression vector, carrying the *E. coli arhR* <br> gene in fusion with C-terminal His-tag | Karaivanova *et al.,* <br> 1999 |

*E. coli* cells were grown by shaking (180 rpm) at 28°C or 37°C on LB media (Miller, 1992) supplemented with appropriate antibiotics: ampicillin 100 $\mu$g/ml, chloramphenicol 25 $\mu$g/ml and kanamycin 25 $\mu$g/ml. For preparation of cell-free extracts, *E. coli* cells were grown at 37°C in a medium with 5.6 g/L, KH$_2$PO$_4$, 28.9 g/L K$_2$HPO$_4$, 10 g/L yeast extract (Difco), 1 mM Mg-acetate, 10g/L glucose, 15 $\mu$g/ml thiamine described by Zubay (Zubay, 1973). *B. stearothermophilus* cells were grown in LB-broth (pH 7.3) at 56°C with vigorous shaking. For *in vivo* expression of His-tagged proteins, recombinant *E. coli* BL21 strains were grown in LB with 100 $\mu$g/ml ampicillin at 28° C until an OD$_{600}$ 0.8-1.0 and after induction by IPTG (1 mM), incubation was continued for 5 h.

**A.2 DNA isolation**

Bacterial biomass of *Thermotoga maritima* MSB8 or *Bacillus stearothermophilus* NCIB8224 was harvested, washed with 10 mM Tris-HCl pH 8.0, 1 mM EDTA, 0.15 M NaCl and thoroughly resuspended in the same solution containing 2 mg/ml lysozyme. After 20 min of incubation at 37°C, a lysis buffer (0.1 M Tris pH 8.0, 1% SDS) was added by gentle mixing and incubation was continued for 30 min at 60°C. The lysis was completed by adding proteinase K (0.6 mg/ml) and incubating the reaction mixture for 30 min at 60°C. After four consecutive extractions by phenol/chloroform/isoamylic alcohol (25:24:1, v/v) DNA was precipitated with 0.6 volume of isopropanol by centrifugation at 15000 rpm for 30 minutes at 4°C, rinsed with 70% ethanol and then diluted in 10 mM Tris-HCl pH 8.0, 0.1 mM EDTA.
Chromosomal and plasmid DNA from *E. coli* strains were isolated according to Ausubel *et al.* (1993).

**A.3 PCR amplification and construction of recombinant DNAs**

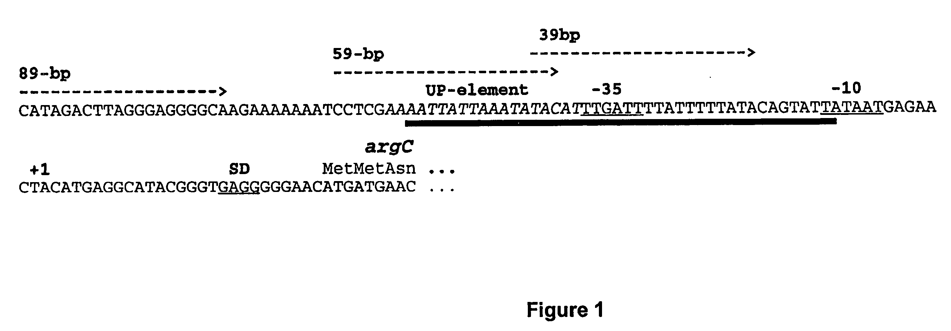

The *B. stearothermophilus PargC* promoter of the *argCJBD* oferon (Sakanyan *et al.,* 1990; Savchenko *et al.,* 1998; Fig. 1) was used as a strong promoter for driving protein synthesis in a cell-free system.
Two oligonucleotide primers were used for amplification of the P*argCo* promoter-operator region and corresponding to the upstream and downstream extremities of said promoter-operator region (5'-CATAGACTTAGGGAGGGGC and 5'-ATGATGATGATGATGATGCATATGTTCCCCCTCACCCGTATG); the latter contains 6 histidine codons to create a N-terminal tag.
Two other oligonucleotides, 5'- CCTCGAAAATTATTAAATATAC and 5'-ACATTTGATTTTATTTTTATAC, were also used to create upstream shortened fragments of promoter sequence, i.e., a 59-bp and a 39-bp fragment of the P*argC* promoter-operator DNA (see also the figure 1).
A DNA sequence coding for a protein of interest was amplified by PCR and fused to the *B. stearothermophilus* P*argC* promoter by the overlap extension method (Ho *et al.,* 1989). Basically, the overlap extension method comprises the following steps:
At the first step, two separate DNA fragments corresponding to N-terminal and C-terminal parts of a given protein, were amplified from chromosomal DNA templates from *Bacillus stearothermophilus* NCIB 8224, *Escherichia coli* K12 XA4, *Thermotoga maritima* MSB8 or *Thermotoga neapolitana* DSM 5068, with specific primers designed in such a way that the amplified products have overlapping sequences with the sequence downstream the P*argC* promoter region.
At a second step, the obtained PCR products were then combined in a subsequent fusion PCR product using only two flanking primers by annealing of the overlapped ends for providing the full-length recombinant DNA template.
All putative genes were fused to a *his*-tag sequence in frame to N-terminal extremity of the open reading frame to enable further purification of the protein by Ni-affinity chromatography as described in A6 hereinafter.
Oligonucleotide primers used for amplification of putative ORFs from *T. maritima* genome are described in the Tables 2A and 2B below.

Table 2A. Oligonucleotide primers used for amplification of putative regulatory genes from *T. maritima*.

| Oligonucleotide primer | Putative protein* | Sequence starting from 5' to 3' |
|---|---|---|
| GntR-0439-His-N-term | GntR 0439 | ATGCATCATCATCATCATCATAAAAAAATCGAAGTGGACCTC |
| Tm0439-GntR-down | -/- | GAACGAAACACCCTCCGCC |
| GntR-0275-His-N-term | GntR 0275 | ATGCATCATCATCATCATCATATCGATGAAATAAAATCTGGAAAG |
| TM-0275-GntR-down | -/- | CTCGCTGGAGGATCACAC |
| GntR-0766-His-N-term | GntR 0766 | ATGCATCATCATCATCATCATTGGTTCAGGATGGATTTTCAC |
| TM-0766-GntR-down | -/- | CAGATAGGTGAACGTGCCG |
| Xyl-0393-His-N-term | XyR 0393 | ATGCATCATCATCATCATCATCCTTCACCGCTTCTTCGAAG |
| TM-0393-XylR-down | -/- | CTTCCAGCCCACCGAAGAGG |
| Xyl-0032-His-N-term | Xyl R-0032 | ATGCATCATCATCATCATCATAAAAAGCTGAATCCAAAATC |
| TM-0032-XylR-down | -/- | CTGATCAGTACCCCATGTG |
| Xyl-0110-His-N-term | Xyl R-0110 | ATGCATCATCATCATCATCATAAATACAACTCACCAAGAATAAAAA |
| TM-0110-XylR-down | -/- | CTCCCGCTTTCTTGAGAAG |
| Xyl-0411-His-N-term | Xyl R-0411 | ATGCATCATCATCATCATCATCTGAATGAAATGGAAAAGAC |
| TM-0411-XylR-down | -/- | CCTGGGTAGAGAATGTAACC |

Table 2B. Oligonucleotide primers used for amplification of genes encoding putative regulatory proteins from *T. maritima*.

| Oligonucleotide primer | Putative protein* | Sequence starting from 5' to 3' |
|---|---|---|
| Xyl-0808-His-N-term | Xyl R-0808 | ATGCATCATCATCATCATCATGGAGGGACCCTTTTGAACAT |
| TM-0808-XylR-down | -/- | CTCGTGATCGGAACTGATGAG |
| Xyl-1224-His-N-term | Xyl R-1224 | ATGCATCATCATCATCATCATCCGAAATCGGTGAGAGCAG |
| TM-1224-XylR-down | -/- | CTCCACGTGTAAATGTACAGTG |
| Lacl-0299-His-N-term | Lacl-0299 | ATGCATCATCATCATCATCATAAAAAAGCTACTCTAAAAGACATTG |
| TM-Lacl-0299-down | -/- | CTATCTCAATCCTTTCACAATG |
| Lacl-0949-His-N-term | Lacl-0949 | ATGCATCATCATCATCATCATAAAAAGGCGCCCTACAATAAATG |
| TM-Lacl-0949-down | -/- | GAAAATCACAGGAGCAAGCG |
| Lacl-1200-His-N-term | Lacl-1200 | ATGCATCATCATCATCATCATAAGAAAAAGTACGTTACGATCAG |
| TM-Lacl-1200-down | -/- | GTAAAATCAAGAATAAGAAAAGG |
| Lacl-1218-His-N-term | Lacl-1218 | ATGCATCATCATCATCATCATGCCTCCATCAAAGATGTGGC |
| TM-Lacl-1218-down | -/- | GCAAACTCGAATTTTTCAGAGG |
| Lacl-1856-His-N-term | Lacl-1856 | ATGCATCATCATCATCATCATCCAACAATAGAAGATGTCG |
| TM-Lacl-1856-down | -/- | GACCACTCGATCTGAACATCC |

\* Oligonucleotide primers were designed from *T. maritima* genome sequence (Nelson *et al.,* 1999).

Oligonucleotide primers used for amplification of operator sequences are described in Table 3.

Table 3. IRD-labelled oligonucleotide primers used for amplification of operator DNA regions

| Oligonucleotides | Operator DNA | Size of operator DNA | Sequence from 5'-extremity |
|---|---|---|---|
| ArgRTn-Up -700 | *argRo T. neapolitana* | 148 bp | IRD700-TGTTACTCTTGAGTTACCAAAAC |
| ArgRTn -down-700 | | | TTATGAGTTCCTGTCTTC-IRD700 |
| GntRo-68UP-800 | *gntKo E. coli* | 68 bp | IRD800-GTCCGGCTGGACAATGTT |

(continued)

| Oligonucleotides | Operator DNA | Size of operator DNA | Sequence from 5'-extremity |
|---|---|---|---|
| GntRo-down-800 | | | GTGGTGCCCCCACAATAC-IRD800 |
| XylRo-104UP-800 | *xylFo E. coli* | 104 bp | IRD800-GGTCATAAATCAAGAAATA AA |
| XylRo-down-800 | | | CACCGCGATAAACGTAACC-IRD800 |
| LacIo-68UP-700 | *lacIo E. coli* | 68 bp | IRD700-GCTTCCGGCTCGTATGT |
| LacIo-down-700 | | | GGTCATAGCTGTTTCCTGTG-IRD700 |
| ArgCo-UP-800 | *argCo B. stearothermophilus* | 100 bp | CTTAGGGAGGGGCAAGAA |
| ArgCo-down-800 | | | CCCGTATGCCTCATGTAG |

If necessary, PCR-produced templates were extracted from agarose gel, treated with phenol-chloroform-isoamyl (25 : 24 : 1, v/v), precipitated with ethanol and resuspended. Their DNA concentration was determined by spectrophotometry and confirmed by comparing band intensity with a Smart Ladder reference DNA (Eurogentec) after SDS-PAGE or agarose electrophoresis.

Otherwise, the quantified PCR-produced DNA templates were directly added to a cell-free extract to drive protein synthesis.

The *E. coli* XA4 *rpoA* gene coding for the $\alpha$ subunit of RNA polymerase was amplified by PCR using oligonucleotide primers 5'-GACA*CCATGG*AGGGTTCTGTGACAGAG (the *Nco*I site is underlined) and 5'-CCG*CTC-GAG*CTCGTCAGCGATGCTTGC (the XhoI site is underlined) and cloned into pET21d(+).

The *E. coli* XA4 *crp* gene coding for CRP was amplified by PCR using oligonucleotide primers 5'-CATG*CCAT-GG*TGCTTGGCAAACC and 5'-CCG*CTCGAG*ACGAGTGCCGTAAACGAC.

The *T. neapolitana groES-groEL* genes coding for chaperonines were amplified by PCR using degenerate oligonucleotide primers 5'-GGAGGGATGGATGATGAARGTNA and 5'-GAYTTYATHGAYCARCAYCCNGA, in which R = A+G, Y = C+T, H = A+C+T, N = all bases. The obtained 2.4 kb DNA fragment was cloned into pCR4-TOPO (Invitrogen). The *T. neapolitana groES* and *groEL* sequences were assigned by the EMBL accession number AF275319.

A chimeric ArgR-EbE protein was constructed by fusing sequences corresponging to DNA-binding and oligomerization domains of *E. coli* ArgR repressor to a linker from *B. stearothermophilus* ArgR. This chimeric sequence displays a better affinity with the heterologous *B. stearothermophilus argCo* operator than with a wild-type *E. coli* ArgR.

The amplified *rpoA, crp,* and wild-type or chimeric *argR* genes were cloned in pET21d(+)vector (Novagen) which allowed expression in frame to a his-tag sequence at the 5'extremity of corresponding proteins. Amplified *T.neapolitana groES* and *groEL* genes were cloned separately in pET19b vector (Novagen) in frame to a *His*-tag at the N-extremity of chaperonine proteins.

Non-labelled and biotine-labelled oligonucleotide primers were purchased from Life Technologies. Dig-labelled and IRD-labelled oligonucleotide primers were purchased from MWG-Biotech.

## A.4 Preparation of cell-free extracts

*E. coli* strains were used for the preparation of cell-free extracts by the method of Zubay (1973) with modifications as follow:

Cells were grown at 37°C, harvested in mid-log phase by centrifugation and washed twice thoroughly in ice-cold buffer containing 10 mM Tris-acetate pH 8.2, 14 mM Mg-acetate, 60 mM KCl, 6 mM $\beta$-mercaptoethanol. Then, cells were resuspended in a buffer containing 10 mM Tris-acetate pH 8.2, 14 mM Mg-acetate, 60 mM KCl, 1 mM dithiotreitol and disrupted by French press (Carver, ICN) at 9 tonnes ($\approx$ 20.000 psi). The disrupted cells were centrifuged at 30.000 g at 4°C for 30 min, the pellet was discarded and the supernatant was centrifuged again. The clear lysate was added in a ratio 1 : 0.3 to the preincubation mixture containing 300 mM Tris-acetate at pH 8.2, 9.2 mM Mg-acetate, 26 mM ATP, 3.2 mM dithiotreitol, 3.2 mM L-amino acids, 3 U/ml pyruvate kinase (Sigma) and incubated at 37°C for 80 min. The mixed extract solution was centrifuged at 6000 g at 4°C for 10 min, dialysed against a buffer containing 10 mM Tris-acetate pH 8.2, 14 mM Mg-acetate, 60 mM K-acetate, 1 mM dithiotreitol at 4°C for 45 min with 2 changes of buffer, concentrated 2-4 times by dialysis against the same buffer with 50% PEG-20.000, followed by additional dialysis without PEG for 1 hour. The obtained cell-free extract was aliquoted and stored at -80°C.

### A.5 Coupled transcription-translation reaction

The expression system was based on the use of transcriptional and translational signals of the *B. stearothermophilus argC* gene (see Fig. 1). The *argC* gene is transcribed from a strong P*argC* promoter which overlaps a 42 bp *argCo* operator (Savchenko *et al.,* 1996) recognized by the *B. stearothermophilus* ArgR repressor (Dion *et al.,* 1997). However, the *E. coli* ArgR repressor has a very weak repression effect with respect to this operator. Therefore, heterologous genes can be overexpressed from *B. stearothermophilus* P*argC* promoter in *E. coli* cells (Savchenko *et al.,* 1998).

T7 promoter based cell-free synthesis was carried out from pET vectors carrying corresponding genes. T7 RNA polymerase was purchased from Promega.

Using the *E. coli* S30 extract system, the transcription-translation coupled reaction was carried out as described by Zubay (1973) with some modifications. The standard pre-mix contained 50 mM Tris-acetate pH 8.2, 46.2 mM K-acetate, 0.8 mM dithiotreitol, 33.7 mM $NH_4$-acetate, 12.5 mM Mg-acetate, 125 $\mu$g/ml tRNA from *E. coli* (Sigma), 6 mM mixture of CTP, GTP and TTP, 5.5 mM ATP, 26.2 mM phosphoenol pyruvate, 8.7 mM $CaCl_2$, 1.9 % PEG-8000, 0.32 mM L-amino acids, 5.4 $\mu$g/ml folic acid, 5.4 $\mu$g/ml FAD, 10.8 $\mu$g/ml NADP, 5.4 $\mu$g/ml pyridoxin, 5.4 $\mu$g/ml para-aminobenzoic acid. In the experiments, wherein pyruvate is used as the energy regenerating compound (Kim and Swartz, 1999), phosphoenol pyruvate is replaced in the standard reaction mixture by 32 mM pyruvate and 6.7 mM K-phosphate pH 7.5, 3.3 mM thiamine pyrophosphate, 0.3 mM FAD and 6 U/ml pyruvate oxidase (Sigma) are added. Typically, to 25 $\mu$l of pre-mix containing all the amino acids except for methionine, 10 $\mu$Ci of [$\alpha^{35}$S]-L-methionine (specific activity 1000 Ci/mmol, 37 TBq/mmol, Amersham-Pharmacia Biotech) and the *E. coli* S30 extracts, 100 - 750 ng of circular plasmid DNA or linear PCR-produced DNA, were added and the mixture was incubated at 37°C for 90-120 min.

If necessary, the protein samples were treated at 80°C for 10 min and then quickly centrifuged. The supernatant was precipitated with acetone and used for protein separation on SDS-PAGE. After coloration with Coomassie Brilliant Blue (Sigma), gels were treated with an amplifier solution (Amersham-Pharmacia Biotech), fixed on a 3 MM paper by vacuum drying and the radioactive bands were visualized by autoradiography using BioMax MR film (Kodak). Molecular weights of putative proteins were deduced from ORFs of the *T. maritima* genome (Nelson *et al.,* 1999) and further confirmed by migration of the corresponding proteins on SDS-PAGE. Quantification of cell-free synthesized proteins (as monomer equivalent) was performed either, (i), by comparing non-radiolabeled protein bands with known reference proteins after coloration with EZBlue gel staining reagent (Sigma) or, (ii), by counting radioactivity of $^{35}$S-labeled protein bands with PhosphorImager.

### A.6 Purification of His-tagged proteins

Recombinant bacteria were suspended in a buffer (50 mM $NaH_2PO_4$ pH 8.0, 300 mM NaCl, 10 mM imidazole), broaken by sonication and His-tagged proteins were purified by affinity chromatography on a Ni-NTA column (Qiagen). The column was washed successively with 10 x bed volumes of the mentioned buffer and proteins were eluted by 6 x bed volumes of elution buffer [50mM Tris-HCl pH 7.9, 50 or 300 mM NaCl, 200 or 400 mM imidazole, 5% (vol/vol) glycerol)] and then dialysed against buffer containing 20 mM $NaH_2PO_4$, pH 8.0, 5 mM $\beta$-mercaptoethanol, 5% (vol/vol) glycerol. Protein concentration was measured by Bradford method (Bradford, 1976).

Proteins synthesized by cell-free synthesis were purified using Ni-NTA-magnetic agarose beads (Quiagen).

### A.7 DNA labelling with IRDyes

Two approaches were used to introduce IRDyes into DNAs. A DNA probe was amplified by PCR using a pair of primers in which one or both primers was labelled by IRD-700 or IRD-800 (see Table 3 above). Alternatively, a DNA probe was subjected to a random incorporation of IRD-700 or IRD-800 using a corresponding kit (LI-COR, Inc.) purchased from ScienceTec.

### A.8 Protein labelling with IRDyes

The purified His-tagged $\alpha$ subunit of *E. coli* RNA polymerase and GroEL of *T. neapolitana* were labelled by FluoroLink Mab Cy 5.5 labelling kit following the manufacturer's instructions (Amersham Pharmacia Biotech). Typically, 5 $\mu$l of a coupling buffer was added to 100 $\mu$l of 0.1 mg/ml protein solution and incubated in the dark for 30 min by shaking every 10 min. The conjugated dye-protein complex was separated from free dye by passing through a PD 10 column and eluted with PBS buffer pH 7.2. Fractions giving a final molar dye ($A_{678}$) /protein ($A_{280}$) ratio around 2 were used for detecting protein-protein interactions.

## A.9 Preparation of protein array

Both, *in vivo* and *in vitro* synthesized protein were used for the preparation of protein array. To produce a sufficient amount of proteins for a small scale analysis of putative ORFs of *T. maritima*, cell-free synthesis was performed in a 200 or 400 $\mu$l reaction mixture for 2 h.

Different quantities of purified proteins (dilutions from 200 pmol to 0.1 pmol) in a A100 buffer (100 mM KCl, 10% glycerol, 20 mM HEPES Na pH 7.9, 0.2 mM EDTA, 10 mM 2-mercaptoethanol and 0.5 mM PMSF) described by Ge (2000) were deposited on a 12 x 8 cm nitrocellulose membrane using a 96-well dot blot apparatus (Bio-Rad) under vacuum and rinsed twice in A100 buffer. Radioactive signals on nitrocellulose membranes were detected by autoradiography using BioMax MS film (Kodak) or PhosphorImager 445 SI (Molecular Dynamics).

## A.10 Detection of intermolecular interactions with fluorescent probes

For protein-DNA interactions, the membranes were incubated in a DNA-binding buffer (DBB): 20 mM Tris-HCl pH 7.9, 50 mM NaCl, 50 mM KCl, 0.1 mM DTT 0.005% surfactant P20 (Biacore) and 25 $\mu$g/ml of a competitor DNA (sonicated salmon DNA) or a mixture of 12.5 $\mu$g/ml poly-dGdC and 12.5 $\mu$g/ml poly-dAdT (Sigma) at room temperature for 30 min. Then, an IRD-labelled DNA probe (10 ng/ml) was added to the prebinding solution and incubated for 12 hours with a slow rotation at different temperatures (4°C, 18°C or 60°C). When necessary, L-arginine (10 mM) or cAMP (5 mM) were included in the reaction mixture. The membranes were washed consecutively with A100 and A500 and then used for the detection of fluorescent signals.

For protein-protein interactions, the membranes were incubated in A100 buffer containing 1% non-fat milk at room temperature for 30 min as described (Ge, 2000). Then, a Cy 5.5-labelled protein probe (5 ng/ml) was added to the prebinding solution and incubated at room temperature for 12 hours.

Alternatively, the protein samples were dialysed against PBS buffer (4.3 mM $Na_2HPO4$, 1.4 mM $KH_2PO4$, 137 mM NaCl, 2.7 mM KCl, pH 7.4 with 20% glycerol) and then deposited on the nitrocellulose membrane under vacuum. After incubation in the PBS solution with 1% BSA for 1 h at room temperature, the membranes were incubated with corresponding probes in the same solution with 0.1% Tween 20 at room temperature for 1 h for detection of protein-protein interactions.

The protein macroarrays were reused after consecutive washing with buffer A containing 1 M $(NH_4)_2SO_4$, 1 M urea (Ge, 2000) and then consecutively with PBS containing 0.1 % Tween 20 and PBS, at room temperature.

To prepare a protein microarray, the method described by MacBeath and Schreiber (MacBeath and Schreiber, 2000) was used with modifications as follow:

The protein samples were dialysed against PBS-buffer with 20% glycerol, and then 600 pl were delivered with a high-precision contact-printing robot SDDC-20 (Virtek) to glass slide covered by superaldehyde (Telechem International), yielding spots of 150 $\mu$m in diameter. The spotted samples were incubated in a humid chamber at room temperature for 3 h to quench the unreacted aldehydes on the slide. After rinsing in the PBS-solution with 1% BSA, the slides were incubated with Cy5.5 labelled probe (final concentration 1 $\mu$g/ml) for 1 h at room temperature.

The protein microarrays were also prepared with an arrayer GMS417 (Affymetrix) by spotting samples on a surface of a BA83 nitrocellulose membrane (Schleich & Schuell).

Near-infrared fluorescence signals on membranes were detected by IR-Imager Odyssey (LI-COR, Inc.).

## A11. Chemiluminescent detection

Diluted samples of proteins in A100 buffer (from 100 pmol to 0.2 pmol) were blotted on nitrocellulose membrane as described above. Incubation was carried out with respect to a biotine-labelled DNA probe (10 ng/ml) and detection of bound DNA-protein complexes was performed with streptavidin (Phototope-star detection kit, BioLabs). In this method, a moderately stable intermediate is formed which spontaneously decays by emitting light at 461 nm. The emission was detected by exposing the membrane to BioMax MS X-ray film (Kodak).

## A.12 Mobility-shift assay (MSA)

DNA binding activity of proteins was also tested by mobility-shift assay using Dig-labelled operator DNAs from *B. stearothermophilus, E. coli, T. maritima* or *T. neapolitana.* DNA binding was performed in DNA-binding buffer in presence of a 100-fold excess of unlabelled sonicated herring sperm DNA and 3 ng Dig-labelled operator DNA at 37°C or 70°C for 30 minutes. If necessary, L-arginine (10 mM) was included in the reaction mixture. Samples were loaded on a 2% agarose gel prepared in TAE buffer (40 mM Tris-base 10 mM sodium acetate, 1 mM EDTA pH 8.0) and migrated by electrophoresis at room temperature at 12 Vcm$^{-1}$ for 1 h. The DNA-protein complexes were transferred onto nylon membranes (Schleicher and Schuell) by capillary method (Ausubel *et al.,* 1993). Immunological detection was carried out with CSPD-mediated luminescence (Boehringer Mannheim). Quantification of free and retarded DNA in gels was done by scanning densitometry of chemioluminograms (Molecular Analyst, Bio-Rad).

**A.13 Surface plasmon resonance (SPR)**

The SPR technique (for a review, see Malmqvist, 1993) was used to study a real-time interaction of wild type and chimeric ArgR proteins with operator DNAs. End-biotinylated DNA fragments corresponding to different operators were prepared by PCR using the above mentioned primers for MSA, except that digoxigenin was replaced by biotin. The labelled DNA fragments were purified from excess of primers and dNTPs by passage through a spin cartridge containing a silica-based membrane (Life Technologies Gibco BRL). Immobilization of biotinylated DNA was carried out on streptavidine-captured biosensor chips SA (Bioacore AB). Biotinylated operator DNA (5 mg/ml) in DNA-binding buffer was injected over the sensor chip at a flow rate of 5 $\mu$l/min at 25°C and its immobilization was controlled manually by achieving nearly 150 RU, a relatively low amount of DNA coupled to the biosensor in order to keep mass transport effects at a minimum. Binding assays were carried out in the same DNA-binding buffer by injection of 10 - 100 nM of a protein (monomer equivalent) at a flow rate 20 $\mu$l/min at 25°C. When necessary, L-arginine (5 mM) or cAMP (5 mM) were included in the reaction mixture. SPR measurements were conducted in parallel channels using a Biacore 3000 (Biacore AB). The sensor chip was regenerated by washing with 1% NaCl at a flow around 10 $\mu$l/min. The channel with no immobilized DNA was used as a reference signal for each cycle. Data for protein-DNA interactions were evaluated from sensorgrams using the 1:1 binding model (BIAevaluation Software Handbook, 1999). The SPR signal is directly proportional to the mass changes at the sensor chip surface and is expressed in resonance units; 1000 RU correspond to a surface concentration of approximately 1 ng / mm$^{-2}$.

**B. Experimental results**

**B.1 Example 1: Preparation of a protein array comprising the binding of cell-free synthesized proteins to nitrocellulose membrane**

In order to prepare an array with chemically non-modified proteins, the method using nitrocellulose membranes as described by Ge (Ge, 2000) was used. However, since the author has not presented data on a possible washing off of membrane-attached proteins, the efficiency of the binding of proteins to nitrocellulose membrane after incubation in binding and washing solutions has been tested.

Two thermostable His-tagged proteins of different size, namely a 25 kDa GntRTm0439 and a 37 kDa LacITm1856, corresponding to putative proteins of *T. maritima* (Nelson *et al.,* 1999) were cell-free synthesized using PCR-produced DNA templates and *E. coli* BL21Z cell-free extracts in 150 $\mu$l reaction mixture for 2 hours. Samples were treated at 80°C for 10 min, centrifuged at 15000 g for 10 min. The pellets were discarded and the proteins were purified from supernatant fractions by affinity chromatography with Ni-NTA-magnetic agarose beads. The incorporated radioactive methionine allowed to quantify the SDS-PAGE separated bands of cell-free synthesized proteins with PhosphorImager. The data were confirmed by the staining of gels with EZBlue.

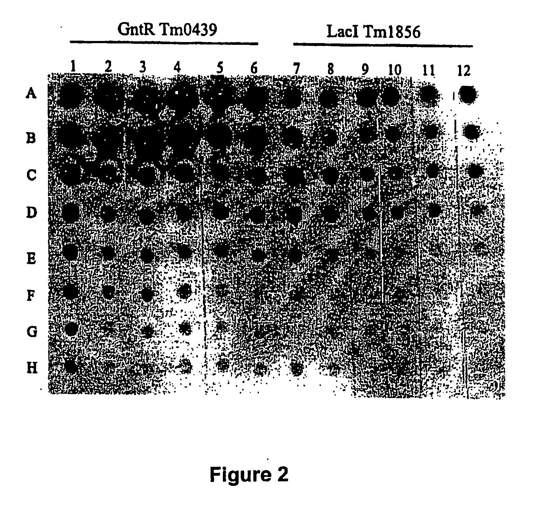

Then, both GntR Tm0439 and LacITm1856 purified proteins were serially diluted and slowly blotted on a nitrocellulose membrane under vacuum for 5 min. Membrane strips corresponding to each line were cut out, incubated in different solutions and then submitted to autoradiography. Practically no diminution of radioactivity was detected in corresponding slots after consecutive thorough washings with A100, A500 and A1000 buffers (as defined in Ge *et al.,* 2000) (Fig. 2; compare samples in horizontal lines for each protein). More than 98% of the two tested proteins, GntR Tm0439 of *T. maritima* and LacI TM1856 of *T. maritima* remained bound on the membranes after the steps of washing. Thus, once a protein is immobilized on the nitrocellulose membrane and rinsed in a binding buffer, it remains perfectly bound to the support.

The binding of cell-free synthesized proteins on a solid phase (here nitrocellulose membrane) that does not involve a covalent chemical linkage appears to be uniform. Moreover, vacuum-blotting cell-free synthesized proteins is advantageous as compared with spotting approaches since both addition and binding of proteins can be done on a relatively large surface, easily controlled even for diluted samples and thereby, quantities of protein patterns in different wells can be normalized precisely.

**B.2 Example 2: Failure of usual chemiluminescent methods for the detection of ArgR protein-DNA interactions**

A 17 kDa ArgR repressor governs arginine biosynthesis in *E. coli* cells (for reviews see Maas, 1994; Glansdorff, 1996). ArgR proteins of thermophilic bacteria *B. stearothermophilus* (Dion *et al.,* 1997; Ni *et al.,* 1999; Karaivanova *et al.,* 1999) and *T. neapolitana* (Dimova *et al.,* 2000) were studied previoulsy. ArgR repressor consists of two structural domains connected by a short linker peptide. The N-terminal domain contains a winged helix-turn-helix module which recognizes and binds *arg*-specific operators (Sunnerhagen *et al.*, 1997; Ni *et al.*, 1999). The C-terminal domain is involved in the formation of trimers which dimerize into hexameric molecules in the presence of L-arginine. Arginine is used as an allosteric co-repressor for improving DNA binding cognate *arg* operators. Sequence and structure similarity between ArgR proteins from various bacteria suggest a common DNA-recognition mechanism. Nevertheless, major differences exist between the different ArgR repressors. In particular, *E. coli* ArgR displays low DNA-binding affinity for the *argCo* operator from *B. stearothermophilus* (Savchenko *et al.,* 1996), whereas both *B. stearothermophilus* and *T. neapolitana* repressors bind *E. coli* operators efficiently (Wang, 1998; Karaivanova *et al.,* 1999; Dimova, 2000).

Taking into consideration this valuable information, interactions of ArgR proteins synthesized *in vivo* or *in vitro,* and then bound to nitrocellulose membrane, were first tested using a chemiluminescent detection method. Recombinant His-tagged ArgR proteins of *B. stearothermophilus* and *T. neapolitana* were synthesized in *E. coli* BL21 (DE3) cells, purified by affinity chromatography and blotted on a membrane at different concentrations. Besides, the *T. neapolitana* ArgR protein was *in vitro* synthesized, purified with Ni-NTA-magnetic agarose beads and blotted on the same membrane. Control samples of S30 extracts were also blotted on the membrane. Biotine or digoxigenin labelled operator DNAs of *B. stearothermophilus argCo* or *T. neapolitana argRo* were used as probes and DNA binding was carried out at 4°C or 60°C in DNA-binding buffer solution overnight. Prewashing, prebinding and binding were carried out in presence of 10 mM L-arginine.

It has been found that a digoxygenine-labelled probe gave a rather weak signal with respect to tested proteins on nitrocellulose membrane, whereas biotine labelled probes gave a strong signal. However, the intensity of the signal was nearly the same for both cell-free extract samples irrespective whether *T. neapolitana* ArgR was synthesized or not (Fig. 3). Moreover, a signal was still strong when binding had been carried out at 60°C.

Although it was possible to distinguish stronger positive signal between samples with *in vitro* synthesized and *in vivo* synthesized ArgR repressor by prolongation of the radioautography exposition, the interpretation of results was always ambiguous. It appeared, that traces of a *E. coli* biotin-binding protein(s), like naturally biotinylated pyruvate carboxylase, were present in samples after purification and these protein traces exhibited high affinity towards streptavidin that mimicked positive signal.

Therefore, these results indicate that the chemiluminescent method is not sensitive enough for testing protein-DNA interactions using the protein arrays comprising cell-free synthesized proteins.

**B.3 <u>Example</u> 3: Detection of ArgR protein-arg operator DNA interactions using near-infrared fluorescent dyes**

Searching for other dyes that could provide a higher signal-to-noise ratio and thereby low background on nitrocellulose membrane, longer-wavelength, near-infrared fluorescent dyes have been tested. A probe consisting of the 59-bp *argCo* operator DNA of *B. stearothermophilus* (see Fig. 1) was labelled by IRD-800. This probe has been used in binding experiments with ArgR repressors blotted on nitrocellulose membrane. Serially 2-fold diluted (from 100 pmole to 0.8 pmole) wild-type ArgR proteins of *T. neapolitana, B. stearothermophilus* as well as a chimeric ArgR-EbE were used to prepare a protein array of a 48-well format. Binding reaction was carried out at 4°C, 18°C or 60°C in presence of 10 mM L-arginine overnight.

As it can be observed in the figure 4a, a weak signal was detected from a chimeric ArgR-EbE with any of the IRD-probes. On the contrary, a strong signal was detected for bound *T. neapolitana* or *B. stearothermophilus* ArgR-DNA complexes. The signal was gradually decreased as a function of the bound protein concentration and the membrane displayed a very low background. A positive signal was observed up to 0.8 pmol and 3.12 pmol, respectively for *T. neapolitana* and *B. stearothermophilus* ArgR. Such a low protein detection level demonstrates that the method is highly sensitive.

Similar results were monitored when binding was performed at three different temperatures and when operator DNA was labelled by IRD-700. It appeared, that the formed complexes between synthesized proteins and IRD-labelled probes were rather stable and that equilibrated state of ArgR-*arg*Co interactions was not affected by temperature.

Next a 39-bp *argCo* operator DNA lacking upstream operator sequence (see Fig. 1) was used as a probe to detect interactions with the ArgR proteins blotted on the membrane. As shown in figure 4b, a binding was detected for higher concentrations of thermostable ArgR proteins, 6.25 pmol and 25 pmol, respectively for *T. neapolitana* and *B. stearothermophilus* repressors.

The affinity of ArgR for arg-specific operator DNA depends on the number of Arg boxes in the operator (Chen *et al.,* 1997), therefore the difference in monitored signals appeared to reflect DNA-binding ability of thermostable repressors to the entire and shortened *argCo* operator sequences.

Data obtained with the protein arrays were confirmed by SPR analysis. Indeed, as shown in figure 5, wild-type ArgR proteins from *T. neapolitana* and *B. stearothermophilus* exhibited a high binding capacity towards the *B. stearothermophilus argCo* operator. In contrast, the ArgR-EbE chimera and especially the wild-type *E. coli* ArgR repressor exhibited a very low binding to this operator DNA. It is worth mentioning that all proteins exhibited a high DNA binding activity with respect to the *E. coli carAB* operator.

Therefore, the data presented in Examples 2 and 3 clearly demonstrate that the protein arrays of the invention can be used in combination with IRD technology

(i) for screening equilibrated protein-DNA interactions; and

(ii) for preliminary quantification of DNA binding properties of proteins.

**B.4 <u>Example 4</u> : Detection of interactions between $\alpha$ subunit of RNA polymerase and *arg* operator DNA**

The transcription level is directed by the strength of binding of the C-terminal domain of $\alpha$ subunit of RNA polymerase to a UP-element in some *E. coli* promoters (Ross et al., 1993; Gourse *et al.*, 2000). The data with a shortened 39-bp probe indicated that a 19 bp AT-rich sequence upstream a -35 site of the P*argC* promoter could make contacts with $\alpha$ subunit of *E. coli* RNA polymerase. To confirm this hypothesis, a $\alpha$ subunit was blotted on the same protein array and tested for interactions with the IRD-labelled *PargC* 59-bp and 39-bp promoter DNAs. Again, as shown in figure 4, a signal was detected for up to 50 pmol of bound $\alpha$ subunit, with respect to a 59-bp operator DNA probe, whereas no binding signal was detected with a 39-bp operator DNA. Thus, these results show that the method of the invention enables to detect binding of $\alpha$ subunit of *E. coli* RNA polymerase to the upstream sequence of the *B. stearothermophilus PargC* promoter.

**B.5 <u>Example 5</u> : Detection of cAMP receptor protein (CRP)-*arg* operator DNA interactions**

*E. coli* CRP is involved in the transcriptional activation of some promoters (Busby and Ebright, 1999). Its binding to DNA requires an allosteric interaction with cAMP (Valentin-Hansen *et al.,* 1996). The sequence upstream the -35 site in *B. stearothermophilus PargCo* promoter-operator shares a weak similarity with CRP binding-sequence of *E. coli* DNA. Therefore, *E. coli* CRP was blotted on the same protein array and tested for binding with *PargCo* DNA probes. As it can be seen in the figure 4, positive signal was detected with a 59-bp but not with a 39-bp operator DNA in presence of 5 mM cAMP. Thus, taken together with the examples 3 and 4, these results demonstrate that the method of the invention is able to detect distinct and specific protein-DNA interactions.

Indeed, these data were confirmed by SPR analysis. As shown in figure 6, CRP displayed a binding to the *B. stearothermophilus PargCo* promoter-operator region in the presence of cAMP, whereas no binding was monitored in the absence of the ligand.

Thus, by using different length IRD-labelled probes and different DNA-binding proteins, it has been shown that the detected protein-DNA interactions by the protein arrays of the invention are specific. The method of the invention permitted to detect relatively low-affinity interactions. This emphasizes the high potential of the method for screening intermolecular interactions at a large scale.

**B.6 <u>Example 6</u> : Detection of protein-protein interactions on a protein array using a probe labelled with near-infrared dyes.**

The transcription efficiency of RNA polymerase holoenzyme can be modulated by interaction of its $\alpha$ subunit with other proteins, like CRP (Gourse *et al.,* 2000). The data presented above showed that *B. stearothermophilus* or *T. neapolitana* ArgR proteins, as well as $\alpha$ subunit of *E. coli* RNA polymerase and CRP have affinity for the same DNA-target, namely the *B. stearothermophilus* P*argCo* promoter-operator region. Therefore, it was presumed that these proteins might contact each other. To verify this hypothesis, a Cy 5.5 labelled $\alpha$ subunit was tested for its binding to other proteins bound to nitrocellulose in a 48-well substractive array format.

Positive signals were detected for *T. neapolitana* and *B. stearothermophilus* ArgR proteins, *E. coli* CRP, but not for *E. coli* ArgR, $\alpha$ subunit of *E. coli* RNA polymerase or *T. neapolitana* GroES and GroEL. As shown in figure 7, protein-protein interactions were detectable for deposited 1.7 pmol (or 0.04 $\mu$g) *E. coli* CRP, 9.1 pmol (or 0.16 $\mu$g) *T. neapolitana* and *B. stearothermophilus* ArgR proteins. The absence of binding signal between $\alpha$ subunits themselves appeared to indicate that dimeric molecules of the protein do not contact together. The negative result with respect to *E. coli* ArgR suggest that this repressor does not bind significantly to the homologous $\alpha$ subunit.

The membrane was washed with a urea-containing buffer A (it was noticed that even thorough washing in this solution does not remove completely bound proteins as judged from residual fluorescence) and reused in a binding reaction with a 39-bp long P*argC* DNA labelled by IRD-800. As expected, specific IRD-800 red signal (IRD-700 gives green colour) was monitored for both *T. neapolitana* and *B. stearothermophilus* ArgR proteins (with a higher affinity for the former), but not for *E. coli* ArgR (Fig. 7b). The specificity of intermolecular interactions was visible also from a weak signal towards *E. coli* CRP (compare Figures 4 and 7), whereas this protein exhibited stronger protein-protein interactions.

Similar data for protein-protein interactions were obtained when the $\alpha$ subunit of RNA polymerase is labelled by IRD-800.

Thus, binding of blotted thermostable ArgRs or *E. coli* CRP to the IRD labelled α subunit of *E. coli* RNA polymerase clearly reflects specific protein-protein interactions. Moreover, these interactions did not prohibit further binding to a IRD labelled 39-bp DNA probe. It appears that the detected protein-protein and protein-DNA interactions are provided by different regions of the blotted proteins.

These data showed that the protein arrays of the invention can be used several times with different probes.

**B.7 Example 7: Screening DNA-protein interactions using a protein array based on cell-free synthesized proteins**

The data presented above showed that both DNA-protein and protein-protein interactions can be detected with IRD-labelled probes of interest with respect to rather low quantities of membrane-blotted proteins purified from bacterial cells. Consequently, the use of IRD-labelled probes has been tested with a protein array prepared with cell-free synthesized proteins.

The optimal conditions were first determined for the different steps of the preparation of the array. These conditions were determined by probing *T. neapolitana* ArgR with respect to IRD-labelled DNA and protein probes.

A $^{35}$S-labelled *T. neapolitana* ArgR has been synthesized in a new PargC-mediated cell-free system using the extracts of the *E. coli* BL21 Star RecBCD (DE3) (pRIL) strain and the His-tagged protein was purified by affinity chromatography by several methods. Each purification step (binding to Ni$^{+2}$, washing and elution with imidazol) was controlled by counting the radioactivity in protein bands after separation in SDS-PAGE. Two purification methods, based on using Ni-coated plates (Pierce) and Ni-NTA-magnetic beads (Quiagen) gave the best results. Since the second method was found more reproducible and with a slightly higher yield, it was used in further experiments for serial purification of His-tagged proteins synthesized *in vitro.*

The purified ArgR (cell-free synthesis was carried out in a 200 μl reaction mixture) was blotted on a nitrocellulose membrane as described in Example 1. Binding was performed with respect to IRD-800-labelled *argRo* of *T. neapolitana* or Cy5.5-labelled α subunit of *E. coli* RNA polymerase, respectively at 60°C and 18°C. In all cases, signals were monitored from up to 4-fold-diluted protein samples, whereas no signal was detected from non-diluted samples without synthesized ArgR. Thus, it was concluded that the thermostable ArgR from *T. neapolitana* is synthesized in cell-free system in a sufficient quantity and a folded protein exhibits specific DNA-protein and protein-protein intereactions as found for a native recombinant protein purified from cells.

Taking into consideration these data, a protein macroarray was prepared with cell-free synthesized proteins for screening DNA-protein and protein-protein interactions in a small scale. 14 ORFs of *T. maritima* coding for putative proteins belonging to Gnt, Xyl and Lac families of regulators (Nelson *et al.*, 1999) were used to synthesize corresponding proteins in a P*argC*-mediated cell-free system. The His-tagged *T. maritima* proteins were purified by affinity chromatography with Ni-NTA-magnetic beads. Quantity of proteins was normalized with respect to each other by counting the radioactivity level in respective bands and taking into account the number of methionine residues in deduced protein sequences. Namely, the following equation has been used:

$$N = R/m \times V,$$

in which

R is a radioactivity level detected in a protein band with PhosphorImager,

m is a number of methionine residues in a monomeric protein,

V is a volume of a sample in μl,

N is a arbitrary quantitity of a synthesized protein.

Practically, the sample with the lowest value N was taken as a reference (the maximal volume) and other protein sample volumes were adjusted (normalized) with respect to the reference (Table 4).

Table 4. Characterization of *T. maritima* putative proteins.

| Putative protein of *T. maritima* | Length of a gene | Deduced molecular weight of a protein | Number of methionine residues |
|---|---|---|---|
| XylR 0393 | 1299 bp | 42.3 kDa | 10 |
| XylR 0032 | 1341 bp | 43.0 kDa | 5 |
| XylR 0110 | 1328 bp | 42.5 kDa | 9 |
| XylR 0411 | 1356 bp | 41.9 kDa | 5 |
| XylR 0808 | 1270 bp | 38.9 kDa | 4 |
| XylR 1224 | 1203 bp | 40.9 kDa | 9 |
| GntR 0275 | 1218 bp | 38.6 kDa | 8 |
| GntR 0766 | 569 bp | 14.0 kDa | 1 |
| GntR 0439 | 843 bp | 25.0 kDa | 4 |
| LacI 0299 | 1235 bp | 38.9 kDa | 7 |
| LacI 0949 | 1194 bp | 37.5 kDa | 8 |
| LacI 1200 | 1109 bp | 38.0 kDa | 9 |
| LacI 1218 | 1184 bp | 36.9 kDa | 5 |
| LacI 1856 | 1186 bp | 37.0 kDa | 8 |

Thus, equimolar quantities of proteins (monomers) were blotted on the nitrocellulose membrane in order to prepare the protein macroarray.

A protein macroarray, prepared as defined above, was divided into three parts and used to detect DNA-protein interactions with respect to three IRD-labelled DNA probes.

Similar DNA-binding sequences are indicative of the resemblance between regulatory systems (for reviews, see Pabo & Sauer, 1992; Roy *et al.*, 1998). Indeed, the presented above data with a ArgR-regulatory system indicated that operator sequences might be conserved in evolutionary distant bacteria *T. neapolitana* and *E. coli*. Therefore, well studied operator sequences from *E. coli* genome were used as probes. Namely, short DNA fragments carrying *gntKo* (Tong *et al.,* 1996), *xylFo* (Song and Park, 1997) and *lacIo* (Oehler *et al.,* 1990) operator sequences were labelled by IRD-800 and probed with respect to the 14 putative regulatory proteins of *T. maritima* (see Table 4) at room temperature.

As shown in figure 8, strong signals were detected from up to 4-fold diluted samples for putative GntTm0766 and GntTm0275 proteins, whereas a signal was rather weak for GntTm0439. Among Lac protein family, a strong signal was detected for LacTm1200, whereas the signal was relatively weak for others. Finally, strong signals were detected for five of six XylTm proteins, except for XylTm0110.

The specificity of the detected signals was confirmed by cross-binding experiment. In particular, GntR proteins could not bind to the IRD-700 labelled *xylFo* operator DNA. Besides, protein array data were confirmed by MSA. In particular, cell-free synthesized GntTm0439, LacTm1856 and probably XylTm0808 bound to the Dig-labelled *E. coli* target operator DNAs by forming retarding bands in agarose gel, as observed also for cell-free synthesized *T. neapolitana* ArgR (Fig. 9).

These results show that protein arrays prepared with cell-free synthesized proteins according to the invention can be used for rapid and parallel screening and characterization of putative DNA-binding proteins, especially transcriptional regulators.

## B.8 Example 8: Screening of protein-protein interactions using array based on cell-free synthesized proteins

A similar protein macroarray bearing cell-free synthesized proteins was used to detect protein-protein interactions using a Cy5.5-labelled α subunit of *E. coli* RNA polymerase as a probe. As shown in Fig. 10, all GntTm proteins exhibit binding with respect to the heterologous α subunit. Three Lac family proteins Tm1856, Tm1218 and Tm0299 also give clear signals from non-diluted samples. A signal was also detected for three Xyl family proteins Tm0393, Tm0808 and Tm0032. A strong response was detected from *E. coli* CRP and *T. neapolitana* ArgR synthesized *in vitro* or *in vivo,* but not from *E. coli* RNA polymerase α subunit or ArgR all used as controls. These data were confirmed with IRD-800 labelled α subunit of RNA polymerase used as a probe.

Thus, protein array prepared with cell-free synthesized proteins are convenient for serial detection of protein-protein interactions such as those involved in transcription regulation using IRD-labelled protein probes.

**B.9 Example 9: Detection of ligand-protein interactions with non-purified proteins from cell homogenates bound to nitrocellulose membrane**

To assess the sensitivity of method of the invention with respect to non-purified proteins bound to nitrocellulose membrane, the following example presents the detection of ligand-protein interactions by applying IRD-labelled secondary antibodies.

The neurotransmitter γ-aminobutyric acid (GABA) recognizes several receptors expressed on cell surface of mammalian cells (Bettler *et al.*, 1998). $GABA_B$ receptor is composed from two subunits $GABA_BR1$ and $GABA_BR2$. $GABA_BR1$ can exist in 2 splice variants, $GABA_BR1a$ and $GABA_BR1b$ that differ by their extracellular part. It was postulated that $GABA_B$ receptor could bind to HNK-1 carbohydrate of adhesion molecules.

To test this hypothesis, the membrane homogenate of cells was used, in which a concomitant expression of rat $GABA_BR1a$ and $GABA_BR2a$ or $GABA_BR1b$ and $GABA_BR2a$ receptors occurred from cloned cDNAs. Membrane homogenates obtained from cells in which the receptors were not expressed (Novartis Pharma), were used as a negative control. Laminine (Sigma), the protein which binds to HNK-1 carbohydrate (Hall *et al.*, 1995) was used as a positive control. Besides, a 8 amino acid peptide with a FLHTRLFV sequence mimicking HNK-1 carbohydrate epitope (Strekalova *et al.,* 2001) was also used as a control.

Serially 2-fold diluted samples of homogenates were arrayed on nitrocellulose membrane under vacuum. A total protein was 6.7 μg in each starting material. The membranes were rinsed twice in A100 buffer solution, blocked with Odyssey blocking buffer for 30 min at 18°C and, after addition of a synthetic HNK-1 carbohydrate (Kornilov *et al.,* 2000) at a final concentration of 12,5 μg/ml or of HNK-1 mimick peptide at a final concentration of 85 μg/ml, incubation was continued overnight. Then, membranes were rinsed in PBS buffer for 5 min, reincubated in blocking buffer for 1 h, incubation was continued for 1 h after addition of rat primary antibodies against HNK-1 carbohydrate (dilution 1:2000) in a blocking buffer with 0.1 % Tween 20. The membranes were washed out in PBS + 0.1% Tween 20 for 5 min and incubated with IRD-800 labelled secondary anti-rat antibodies (dilution 1:2000 in blocking buffer) for 1 h. The membranes were consecutively washed with PBS + 0.1% Tween 20 for 5 min and then in PBS for another 5 min.

In a control experiment, the membrane with immobilized homogenates of cells with expressed $GABA_B$ R1a and $GABA_B$ R1b and $GABA_B$ R2a receptor subunits were directly incubated with rat $GABA_B$ antibodies (dilution 1:2000) and then with IRD-800-labelled secondary antibodies as described above.

Figure 11 reveals that a strong signal was detected up to 128-fold diluted homogenate samples with expressed GABA receptors, whereas only a weak signal was detected for control homogenate from non-diluted and 2-fold diluted samples. Thus, IRD-technology provides a rather high sensitivity sufficient to detect minor quantities of target proteins in cell membrane homogenates and can be used with non-purified protein samples.

**B.10 Example 10: Detection of DNA-protein interactions in crude cell extracts blotted on a nitrocellulose membrane**

Another experiment was also carried out to evaluate the sensitivity of IRD-detection method for DNA-protein interactions in crude cell extracts. The design of this experiment was based on the observation that a shortened 39-bp P*argCo* operator DNA of *B. stearothermophilus* exhibited a specific affinity with respect to *B. stearothermophilus* and *T. neapolitana* ArgR proteins, whereas no binding could be detected with respect to homologous *E. coli* ArgR and α subunit of RNA polymerase or a weak binding is detected for CRP (see Example 3). Therefore, such a short operator sequence could be used as a probe for screening a total protein pool in *E. coli* extracts in order to detect DNA-protein interactions with respect to *B. stearothermophilus* or *T. neapolitana* ArgR repressors synthesized in *E. coli* cells.

The *E. coli* BL21 (DE3) cells bearing a pETd-ArgR-His plasmid with corresponding *E. coli*, *B. stearothermophilus* or *T. neapolitana argR* gene were cultivated up to $OD_{600}$ 0.8, then 1 mM IPTG was added and cultivation was continued for 5 h. Samples were taken before and after 1 and 5 h IPTG-induction, thoroughly washed three times in A100, sonicated and centrifuged. After discarding the pellet, the supernatant was 2-fold serially diluted in A100 and blotted on the nitrocellulose membrane under vacuum. The membrane was probed with a IRD-800 labelled 39-bp P*argCo* DNA at room temperature overnight. As it is shown in Figure 12, it was found, that starting from 1 h IPTG-induction samples with expressed *B. stearothermophilus* or *T. neapolitana* ArgR provided strong signal but not those with *E. coli* repressor.

The three ArgR proteins were also synthesized in a cell-free system. The reaction mixture was blotted to a nitrocellulose membrane and probed with respect to a IRD-800 labelled 39-bp P*argCo* DNA probe at room temperature overnight. Signals were detected for samples with synthesized ArgR proteins of *B. stearathermophilus* and *T. neapolitana,* whereas no signal was detected for *E. coli* ArgR repressor.

Thus, the method of the invention is also sensitive for the detection of specific DNA-protein interactions with respect to non-purified proteins in crude cell extracts, especially when a high amount of protein targets is synthesized.

### B. 11 Example 11: Detection of protein-protein interactions on a glass slide using near-infrared fluorescence detection

Similar experiments were performed with IRD-labeled proteins chemically linked to superaldehyde-covered glass slide.

It has been found that protein-protein interactions can be detected with samples of cell-free synthesized proteins spotted on slides in spite of relatively high background. In particular, signals were monitored from spotted several proteins, namely for *T. neapolitana* ArgR cell-free synthesized and for *B. stearothermophilus* ArgR, *T. neapolitana* GroEs and *E. coli* CRP synthesized *in vivo* with respect to the Cy5.5- labelled GroEL of *T. neapolitana* (Fig. 13). The highest sensitivity was detected for CRP protein, up to 2.3 pg of protein from 256-fofd diluted sample. Signals were not monitored for *E. coli* ArgR and $\alpha$ subunit of RNA polymerase.

Thus, these preliminary data indicate that superaldehyde-covered glass slides (or preferably nitrocellulose membrane slides) can also be used as convenient supports for fabrication of protein microarrays from cell-free synthesized proteins and following detection of protein-protein interactions with IRDyes according to the invention.

### B. 12 Example 12: Detection of DNA-protein interactions using crude cell extracts directly deposited as a microarray

Another experiment was carried out to evaluate the sensitivity of the array method using IRDyes for analysis of DNA-protein interactions using crude cell extracts being spotted directly on a nitrocellulose membrane by an arrayer Affymetrix GMS417.

First, expression of *E. coli, B. stearothermophilus* or *T. neapolitana arg* genes, or *E. coli rpoA* or crp genes using the extracts of non-induced or IPTG-induced *E. coli* BL21 (DE3) cells was analyzed from a T7 phage promoter by SDS-PAGE (Fig. 14; Fig. 15). The three *E. coli* ArgR, RpoA and CRP overexpressed proteins started being visible as clear bands after 30 min induction whereas thermophilic proteins appeared only after 120 min induction.

The crude cell extracts were serially 2-fold diluted, spotted on duplicate nitrocellulose membranes and incubated with 59-bp or 39-bp IRDyes-800 labelled DNA fragments. The same membranes contained spotted purified proteins (lower lines). No signal was detected in non-induced bacteria harboring the cloned *argR* or *rpoA* genes when probed to a 59-bp DNA. The fluorescent signal was not detected from IPTG-induced cell extracts carrying the overexpressed *E. coli argR* gene. However, a strong signal was detected after 30 min induction of *B. stearothermophilus* or *T. neapolitana* ArgR repressors (Fig. 14 e and f), though no protein bands were yet visible in extracts by SDS-PAGE. Fluorescent emission was also monitored for the overexpressed *E. coli* RNA polymerase $\alpha$ subunit spots but only after 60 min induction (Fig. 15 c) that was accompanied by the appearing an abondant band of the protein on a polyacrylamid gel. Surprisingly, signal was detected from extracts of non-induced and 2-fold diluted extracts with CRP (Fig. 15 d) indicating the presence of a pool of a non yet identified putative protein(s) in *E. coli* cells which possesses a good affinity for the P*argCo* promoter-operator region in the presence of cAMP. However, even in this case a clear signal appeared in more diluted samples after 120 min induction.

When spotted extracts were tested with respect to a shorter 39-bp DNA probe the fluorescent response was still strong for *B. stearothermophilus* and especially, for *T. neapolitana* ArgR repressors (Fig. 14 h and i). The signal was shifted two spots to the lower diluted samples of the *B. stearothermophilus* repressor thereby confirming the worse recognition of a single Arg box than a double box. Though weaker but obvious signals were detected for the same dilutions of the spotted *T. neapolitana* ArgR protein as compared to binding to a 59-bp DNA probe thus proving its ability to recognize a single and double Arg box carrying *argCo* operator with almost similar affinity.

The appearance of fluorescent signals from spotted crude cell extracts strongly depended on the amount of a target proteins and their DNA-binding affinity as observed on arrays with purified proteins (lower lines in Fig. 14 and 15).

These data show that the use of IRDyes permits a very sensitive detection and allow avoiding a step of purification which is generally considered to be necessary for protein arrays analysis.

### B. 13 Example 13: Detection of protein-protein interactions with the array method

The sensitivity of the protein array method using IRDyes is also validated by the next example for analysis of protein-protein interactions in crude cell extracts spotted on a nitrocellulose membrane.

The *E. coli* RNA polymerase $\alpha$ subunit was labeled with Cy5.5 and probed to the three ArgR repressors, CRP or $\alpha$ subunit itself, all spotted as crude cell extracts. The same membranes also contained purified proteins (lower lines).

Signals were monitored from all samples of non-diluted and 2- and 4-fold diluted crude extracts, up to 60 pg of total protein spots suggesting that the *E. coli* RNA polymerase $\alpha$ subunit displays rather stable contacts with many proteins that causes a background fluorescence and prohibits the detection of specific interactions. Nevertheless, a gradual raising of a signal intensity was monitored in extracts with CRP as a function of the amount of the oversynthesized protein after IPTG induction (compare horizontal lines in Fig. 15 f). From purified *E. coli* CRP spots the detected signal corresponded to to 600 amol of the spotted protein (Fig. 15 f). The interaction between *E. coli* RNA polymerase α subunit and CRP has already been demonstrated (Busby and Enright, 1994).

Clear signals were monitored from purified *B. stearothermophilus* and *T. neapolitana* repressor spots (up to 3.3 fmol and 825 amol, respectively) whereas no binding was detected with respect to *E. coli* ArgR (Fig. 14 j, k and i). Thus, the *E. coli* RNA polymerase α subunit possesses a capacity to interact with heterologous thermophilic repressors but not with a homologous ArgR, at least under the conditions used.

In order to verify the array data the fluorescence anisotropy of protein interactions was measured by adding CRP or the either of the three ArgR repressors to the binding solution with the *E. coli* Cy3-labelled RNA polymerase α subunit. The anisotropy shape was found to be similar for CRP - RNA polymerase α subunit interactions irrespective of the addition of cAMP into a buffer and a calculated apparent dissociation constant Kd was 300 nM close to 280 nM determined by Heyduck and collaborators at similar conditions (Heyduck *et al.,* 1993). Both *B. stearothermophilus* and *T. neapolitana* repressors, but not *E. coli* ArgR, also displayed a typical saturated anisotropy shape and Kd values indicated a rather good affinity between thermophilic ArgRs and a mesophilic RNA polymerase α subunit (Table 5). Arginine did not affect this binding.

Table 5. Evaluation of protein -protein interactions by fluorescence anisotropy.

| Protein/conditions* | Kd (M, monomer) |
|---|---|
| **ArgR *B. stearothermophilus*** | |
| - arginine | 16 nM |
| + arginine | 16 nM |
| **Arg R *T. neapolitana*** | |
| - arginine | 60 nM |
| + arginine | 60 nM |
| **B.11 ArgR *E. coli*** | no interaction |
| **CRP *E. coli*** | |
| - cAMP | 300 nM |
| + cAMP | 300 nM |
| * Assays were carried out in PBS containing 0.3 M NaCl in the absence or in the presence of 10 mM arginine or 0.5 mM cAMP | |

Thus, the supplied methods allow to detect moderate force protein-protein interactions (of the order $10^{-7}$ M) both with spotted purified samples and crude cell extracts that emphasizes their extreme sensitivity that might be important for new applications of array technologies.

The innovations introduced in this work allowed to exclude fastidious steps of gene cloning and protein expression in living cells in order to prepare protein arrays for detection of diverse intermolecular interactions, as well as to eliminate $^{32}$P-labelled radioactive probes. Another advantage of cell-free synthesized proteins application for manufacturing of protein arrays is a possibility to perform accurate normalization of samples to be immobilized on a solid phase. In this study, it was achieved an extremely high sensitivity of the detection of DNA-protein, protein-protein and ligand-protein interactions by applying IRDyes. The new designed cell-free protein production system along with the used IRDyes can be applied as a universal, sensitive, less time-consuming and low-expensive protein array method for identification of intermolecular interactions.

REFERENCES

Ausubel, F.M., Brent, R., Kingston, R. E., Moore, D. D., Seidman, J. G., Smith, J. A. & Struhl, K. (1993). *Current Protocols in Molecular Biology.* John Wiley & Sons, N. Y.

Bettler, B., Kaupmann, K. & Bowery, N. (1998). GABA$_B$ receptors: drugs meet clones. *Cur. Opinion Neurobiol.* **8**, 345-350.

Bradford, M.M. (1976). A rapid sensitive method for the quantitation of microgram quantities of protein utilising the principle of protein-dye binding. *Anal. Biochem.* **72**, 248-254.

Busby, S. & Ebright, R. E. (1999). Transcription activation by catabolite activator protein (CAP). *J. Mol. Biol.* **293,** 199-213.

Büssow, K., Cahill, D., Nietfeld, W., Bancroft, D., Scherzinger, E., Lehrach, H. & Walter, G. (1998). A method for global protein expression and antibody screening on high-density filters of an arrayed cDNA library. *Nucleic. Acids* Res. **26**, 5007-5008.

Chen, S.-H., Merican, A. F. & Sherratt, D. J. (1997). DNA binding of *Escherichia coli* arginine repressor mutants altered in oligomeric state. *Mol. Microbiol.* **24**, 1143-1156.

Dimova, D. (2000). La protéine régulatrice ArgR de bactéries hyperthermophiles du genre *Thermotoga*: clonage, thermostabilité et interaction avec l'ADN. Ph. Thesis, Nantes.

Dimova, D., Weigel, P., Takahashi, M., Marc, F., Van Duyne, G. & Sakanyan, V. (2000). Thermostability, oligomerization and DNA binding properties of the regulatory protein ArgR from the hyperthermophilic bacterium *Thermotoga neapolitana. Mol. Gen. Genet.* **263**, 119-130.

Dion, M., Charlier, D., Wang, H., Gigot, D., Savchenko, A., Hallet, J.-N., Glansdorff, N. & Sakanyan, V. (1997). The highly thermostable arginine repressor of *Bacillus stearothermophilus*: gene cloning and repressor-operator interactions. *Mol. Microbiol.* **25,** 385-398.

Ebright, R. E. & Busby, S. (1995). The *Escherichia coli* RNA polymerase alpha subunit: structure and function. *Curr. Opin. Genet. Dev.* **5,** 197-203.

Esser, M. M., Urlacher, T. M., Draney, D. & Olive, D. M. (2000). Novel applications for infrared fluorescence imaging. On-line posters, www.licor.com

Ge, H. (2000). UPA, a universal protein assay system for quantitative detection of protein-protein, protein-DNA, protein-RNA and protein-ligand interactions. *Nucleic Acids Res.* **28**, e3

Glansdorff, N. (1996). Biosynthesis of arginine and polyamines. In: Neidhardt, F. C. (Ed. in chief) *Escherichia coli* and *Salmonella*: cellular and molecular biology. American Society for Microbiology Press, Washington, DC, p. 408-433.

Gourse, R. L., Ross, W. & Gaal, T. (2000). Ups and downs in bacterial transcription initiation: the role of the alpha subunit of RNA polymerase in promoter recognition. *Mol. Microbiol.* **37**, 687-695.

Hall, H., Vorherr, T. & Schachner, M. (1995). Characterization of a 21 amino acid peptide sequence of the laminin G2 domain that is involved in HNK-1 carbohydrate bindning and cell adhesion. *Glycobiology* **5**, 435-441.

Holt, L. J., Büssow, K., Walter, G. & Tomlinson, M. (2000). By-passing selection: direct screening for antibody-antigen interactions using protein arrays. *Nuclic Acids Res.* **28**, e72.

Joung, J. K., Ramm, E. I. & Pabo, C. O. (2000). A bacterial two-hybrid selection system for studying protein-DNA and protein-protein interactions. *Proc. Natl. Acad. Sci. USA* **97,** 7382-7387.

Karaivanova, I., Weigel, P., Takahashi, M., Fort, C., Versavaud, A., Van Duyne, G., Charlier, D., Hallet, J.-N., Glansdorff, N. & Sakanyan, V. (1999). Mutational analysis of the thermostable arginine repressor from *Bacillus stearothermophilus*: dissecting residues involved in DNA binding properties. J. *Mol. Biol.* **291,** 843-855.

Kigawa, T., Yabuki, T., Yoshida, Y., Tsutsui, M., Ito, Y., Shibata, T. & Yokoyama, S. (1999). Cell-free production and stable-isotope labeling of milligram quantities of proteins. *FEBS Letters* **442**, 15-19.

Kim, D.-M. & Swartz, J. R. (1999). Prolonging cell-free protein synthesis with a novel ATP regeneration system. *Biotech.* & *Bioengin.* **66**, 180-188.

King, R. W., Lustig, K. D., Stukenberg, P. T., McGarry, T. J. & Kirschner, M. W. (1997). Expression cloning in the test tube. *Science* **277**, 973-974.

Kornilov, A. V., Sherman, A. A., Kononov, L. O., Shashkov, A. S. & Nifant'ev, N. E. (2000). Synthesis of 3-O-sulfoglucuronyl lacto-N-neotetraose 2-aminoethyl glycoside and biotinylated neoglycoconjugates. *Carbohydr Res.* **329**, 717-730.

Lesley, S. S., Brow, M. A. & Burgess, R. R. (1991). Use of *in vitro* protein synthesis from polymerase chain reaction-generated templates to study interaction of *Escherichia coli* transcription factors with core RNA polymerase and for epitope mapping of monoclonal antibodies. *J. Biol. Chem,* **266**, 2632-2638.

Lustig, K. D., Stukenberg, P. T., McGarry, T. J., King, R. W., Cryns, V. L., Mead, P. E., Zon, L.I., Yuan, J. & Kirschner, M. W. (1997). Small pool expression screening: identification of genes involved in cell cycle control, apoptosis, and early development. *Methods Enzymol.* **283**, 83-99.

Maas, W. (1994). The arginine repressor of *Escherichia coli. Microbiol. Rev.* **58**, 631-640.

MacBeath, G. & Schreiber, S. L. (2000). Printing proteins as microarrays for high-throughout function determination. Science **289**, 1760-1763.

Mahlknecht, U., Ottmann, O. G. & Hoelzer, D. (2001). Far-Western based protein-protein interaction screening of high-density protein filter arrays. *J. Biotechnol.* **88**, 89-94.

Malmqvist, M. (1993). Biospecific interaction analysis using biosensor technology. *Nature* **361**, 186-187.

Miller, J. H. (1992). A short course in bacterial genetics. Cold Spring Harbor, NY: Cold Spring Harbor Laboratory.

Nelson, K. E. *et al.* (1999). Evidence for lateral gene transfer between Archaea and Bacteria from genome sequence of *Thermotoga maritima. Nature* **399**, 323-329.

Ni, J.-P., Sakanyan, V., Charlier, D., Glansdorff, N. & Van Duyne, G. D. (1999). Structure of the arginine repressor from *Bacillus stearothermophilus. Nature Struct. Biol.* **6,** 427-432.

Oehler, S., Eisman, E., Krämer, H. & Müller-Hill, B. (1990). The three operators of the *lac* operon cooperate in repression. *EMBO J.* **9,** 973-979.

Pabo, C. O. & Sauer, R. T. (1992). Transcription factors: structural families and principles of DNA recognition. *Annu. Rev. Biochem.* **61**, 1053-1095.

Pelham, H. R. & Jackson, R. J. (1976). An efficient mRNA-dependent translation system from reticulocyte lysates. *Eur. J. Biochem.* **677,** 247-256.

Roberts, B. E. & Paterson, B. M. (1973). Efficient translation of tobacco mosaic virus RNA and rabbit globin 9S RNA in a cell-free system from commercial wheat germ. *Proc. Natl. Acad. Sci. USA* **70,** 2330-2334.

Ross, W., Gosink, K. K., Salomon, J., Igarashi, K., Zou, C., Ishihama, A., Severinov, K. & Gourse, R. L. (1993). A third recognition element in bacterial promoters: DNA binding by the $\alpha$ subunit of RNA polymerase. *Science* **262**, 1407-1413.

Roy, S., Garges, S. & Adhya, S. (1998). Activation and repression of transcription by differential contact: two sides of a coin. *J. Biol. Chem.* **273**, 14059-14062.

Sakanyan, V. A., Hovsepyan, A. S., Mett, I. L., Kochikyan, A. V. & Petrosyan, P. K. (1990). Molecular cloning and structural-functional analysis of arginine biosynthesis genes of the thermophilic bacterium *Bacillus stearothermophilus. Genetika* (USSR) **26,** 1915-1925.

Savchenko, A., Charlier, D., Dion, M., Weigel, P., Hallet, J.-N., Holtham, C., Baumberg, S., Glansdorff, N. & Sakanyan, V. (1996). The arginine operon of *Bacillus stearothermophilus*: characterization of the control region and its interaction with the heterologous *B. subtilis* arginine repressor. *Mol. Gen. Genet.* **252**, 69-78.

Savchenko A., Weigel P., Dimova D., Lecocq M. & Sakanyan V. (1998). The *Bacillus stearothermophilus argCJBD* operon harbours a strong promoter as evaluated in *Escherichia coli* cells. *Gene* **212**, 167-177.

Schutz, A. R., Altschuler, Y., Mostov, K. E. & Olive, D. M. (2000). Highly sensitive detection of proteins on membranes with near-infrared fluorescence. On-line posters, www.licor.com

Song, S. & Park, C. (1997). Organization and regulation of the D-xylose operons in *Escherichia coli* K-12: XylR acts as a transcriptional activator. *J. Bacteriol.* **179,** 7025-7032.

Strekalova, T., Wotjak, C. T. & Schachner, M. (2001). Intrahippocampal Administration of an Antibody against the HNK-1 Carbohydrate Impairs Memory Consolidation in an Inhibitory Learning Task in Mice. *Mol Cell Neurosci.* **17,** 1102-1113.

Sunnerhagen, M., Nilges, M., Otting, G. & Carey, J. (1997). Solution structure of the DNA binding domain and model for the complex multifunctional hexameric arginine repressor with DNA. *Nature Struct. Biol.* **4**, 819-826.

Tong, S., Porco, A., Isturiz T., & Conway T. (1996). Cloning and molecular genetic characterization of the *Escherichia coli gntR, gntK,* and *gntU* genes of GntI, the main system for gluconate metabolism. *J. Bacteriol.* **178**, 3260-3269.

Valentin-Hansen, P., Sogaard-Andersen. L. & Pedersen, H. (1996). A flexible partnership: the CytR anti-activator and the cAMP-CRP activator protein, comrades in transcriptional control. *Mol. Microbiol.* **20**, 461-466.

van Essen, A. J., Kneppers, A. L., van der Hout, A. H., Scheffer, H., Ginjaar, I. B., ten Kate, L. P., van Ommen, G. J., Buys, C. H. & Bakker, E. (1997). The clinical and molecular genetic approach to Duchenne and Becker muscular distrophy: an updated protocol. *J. Meth. Genet.* **34,** 805-812.

Vidal, M. & Legrain, P. (1999). Yeast forward and reverse "n"-hybrid systems. *Nucleic Acids Res.* **27**, 919-929.

Wang, H., Glansdorff, N. & Charlier, D. (1998). The arginine repressor of *Escherichia coli* K-12 makes direct contacts to minor and major groove determinants of the operators. *J. Mol. Biol.* **277,** 805-824.

Zubay, G. (1973). *In vitro* synthesis of protein in microbial systems. *Ann. Rev. Genet.* **7**, 267-287.

**Claims**

1. A method for the detection of intermolecular interactions between probe(s) and protein targets, said protein targets, preferably including at least one protein or polypeptide unproducible *in vivo* which, when expressed *in vivo* in host cells, is toxic for said host cells or cannot be purified, or does not fold correctly, said method combining the steps of:

   a) providing protein targets by serially *in vitro* synthesizing them, using a cell-free protein synthesis method with an excess of the α subunit of RNA-polymerase compared to other subunits of the like;
   b) binding to a support at defined positions, said protein targets to prepare a protein array ;
   c) providing probe(s) labelled with a near-infrared fluorescent dye,
   d) contacting said protein array with the labelled probe(s) in a medium suitable to allow a specific binding, and
   e) detecting on the protein array, the protein target(s) to which the labelled probe(s) specifically binds using an Infrared imager device.

2. The method according to Claim 1, wherein said cell-free protein synthesis method comprises the use of a cell-free extract, preferentially a bacterial cell-free extract, preferably *E. coli* cell-free extract.

3. The method according to Claim 1 or 2, wherein a cell-free protein synthesis system comprises the use of a PCR-produced DNA template, said PCR-produced DNA template having an Open Reading Frame encoding said protein or polypeptide, and an additional DNA sequence which is at least 3 bp long, preferably longer than 100 bp, and more preferably longer than 200 bp, located immediately downstream the stop codon of the Open Reading Frame encoding said protein or polypeptide.

4. The method according to any of Claims 1 to 3, wherein said DNA template comprises a strong promoter with at least one UP element, localized upstream of the ORF.

5. The method according to Claims 2 to 4, wherein said RNA-polymerase is a purified thermostable polymerase, preferably from *T. thermophilus.*

6. The method according to any of Claims 1 to 5, wherein said probe molecule is a nucleic acid, a polypeptide or a protein labelled with a near-infrared fluorescent dye.

7. The method according to any of Claims 1 to 6, wherein said synthesized proteins or polypeptides are not purified prior to binding to the support.

8. The method according to any of Claims 1 to 7, **characterized in that** it further comprises steps for normalization of the amount of synthesized protein prior to binding to a support, said normalization steps consisting of:

   a) labelling each synthesized protein, preferably by supplying a radioactive amino acid in the cell-free system,
   b) quantifying each labelled protein, and
   c) providing equivalent amount of each synthesized protein prior to binding to a support for normalization.

9. The method according to any of claims 1 to 8, wherein at least two different probes are used, said probes being labelled with IRDyes of different wave-length.

10. The method according to any of claims 1 to 9, wherein said near infrared fluorescent dyes have wave length superior than 680 nm.

11. A method for the preparation of a protein array comprising protein targets, preferably including one protein or polypeptide unproducible *in vivo* which, when expressed *in vivo* into host cells, is toxic for said host cells or cannot be purified, or does not fold correctly, said method comprising:

   a) synthesizing in parallel protein targets using a cell-free protein synthesis method with an excess of the α subunit of RNA-polymerase according to other subunits of the like;
   b) recovering the synthesized proteins or polypeptides; and,
   c) preparing a protein array by binding synthesized proteins or polypeptides to a support at defined positions.

12. The method according to Claim 11, wherein said cell-free protein synthesis method comprises the use of a bacterial cell-free extract, preferably *E. coli* cell-free extract.

13. The method according to Claim 11 or 12, wherein said cell free protein synthesis method comprises the use of a PCR-produced DNA template, said PCR-produced DNA template comprising the Open Reading Frame encoding said protein or polypeptide, and an additional DNA sequence which is at least 3 bp-long, preferably longer than 100 bp, and more preferably longer than 200 bp, located immediately downstream the stop codon of said Open Reading Frame encoding said protein or polypeptide.

14. The method according to any of Claims 11 to 13, wherein said DNA template comprises a strong promoter with at least one UP element, localized upstream of the ORF.

15. The method according to Claims 12 to 14, wherein a purified thermostable RNA polymerase, preferably from *T. thermophilus,* is added into the bacterial cell-free extract.

16. The method according to any of Claims 11 to 15, wherein said synthesized proteins or polypeptides are not purified prior to binding to the support.

17. Use of target proteins for the preparation of protein arrays, wherein said target proteins are deposited on the array as a crude extract, without purification after their synthesis obtained by a cell-free protein synthesis system with an excess of the α subunit of RNA-polymerase compared to other subunits of the like.

18. The use according to Claim 17, wherein said cell-free protein synthesis system comprises a bacterial cell-free extract, preferably *E. coli* cell-free extract.

19. The use according to Claim 18, wherein said proteins or polypeptides contained in the crude extract are synthesized from a PCR-produced DNA template, said PCR-produced DNA template comprising the Open Reading Frame encoding said protein or polypeptide, and an additional sequence which is at least 3 bp long, preferably longer than 100 bp, and more preferably longer than 200 bp, located immediately downstream the stop codon of the gene encoding said protein or polypeptide.

20. The use according to any of Claims 17 to 19, wherein said DNA template DNA template comprises a strong promoter with at least one UP element, localized upstream of the ORF.

21. The use according to Claims 17 to 20, wherein a purified thermostable RNA polymerase, preferably from *T. thermophilus,* is added into the bacterial cell-free extract.

22. The use according to Claim 17 to 21, wherein, at least one of the proteins or polypeptides, when expressed *in vivo* into host cells, is toxic for said host cells or cannot be purified, or does not fold correctly

## Patentansprüche

1. Verfahren zum Nachweis von intermolekularen Wechselwirkungen zwischen Sonde(n) und Proteinzielen, wobei die Proteinziele vorzugsweise mindestens ein *in vivo* nichtherstellbares Protein oder Polypeptid, das bei Expression *in vivo* in Wirtszellen toxisch für die Wirtszellen ist oder nicht gereinigt werden kann oder sich nicht korrekt faltet, einschließen, wobei das Verfahren die folgenden Schritte kombiniert:

   a) Vorsehen von Proteinzielen durch serielles *in vitro*-Synthetisieren dieser unter Verwendung eines zellfreien Proteinsyntheseverfahrens mit einem Überschuss der α-Untereinheit von RNA-Polymerase im Vergleich zu anderen Untereinheiten derselben;
   b) Binden der Proteinziele an einen Träger an definierten Positionen, um einen Protein-Array herzustellen,
   c) Vorsehen von Sonde(n), die mit einem Nah-Infrarot-Fluoreszenzfarbstoff markiert sind,
   d) Kontaktieren des Protein-Arrays mit den markierten Sonde(n) in einem Medium, das zum Ermöglichen einer spezifischen Bindung geeignet ist, und
   e) Nachweisen, auf dem Protein-Array, der Proteinziel(e), an welche(n) die markierten Sonde(n) spezifisch bindet bzw. binden, wobei eine Infrarot-Bilderzeugungsvorrichtung angewandt wird.

2. Verfahren nach Anspruch 1, wobei das zellfreie Proteinsyntheseverfahren die Verwendung eines zellfreien Extraktes, vorzugsweise eines bakteriellen zellfreien Extraktes, bevorzugt eines zellfreien *E.coli*-Extraktes, umfasst.

3. Verfahren nach Anspruch 1 oder 2, wobei ein zellfreies Proteinsynthesesystem die Verwendung einer durch PCR erzeugten DNA-Matrize umfasst, wobei die PCR-erzeugte DNA-Matrize einen das Protein oder Polypeptid codierenden offenen Leserahmen und eine zusätzliche DNA-Sequenz, welche mindestens 3 bp lang, vorzugsweise länger als 100 bp und weiter bevorzugt länger als 200 bp ist, die unmittelbar stromabwärts des Stop-Codons des für das Protein oder Polypeptid codierenden offenen Leserahmens lokalisiert ist, aufweist.

4. Verfahren nach mindestens einem der Ansprüche 1 bis 3, wobei die DNA-Matrize einen starken Promotor mit mindestens einem UP-Element, der stromaufwärts des ORF lokalisiert ist, umfasst.

5. Verfahren gemäß den Ansprüchen 2 bis 4, wobei die RNA-Polymerase eine gereinigte thermostabile Polymerase, vorzugsweise aus *T. thermophilus,* ist.

6. Verfahren nach mindestens einem der Ansprüche 1 bis 5, wobei das Sondenmolekül eine Nukleinsäure, ein Polypeptid oder ein Protein ist, das mit einem Nah-Infrarot-Fluoreszenzfarbstoff markiert ist.

7. Verfahren nach mindestens einem der Ansprüche 1 bis 6, wobei die synthetisierten Proteine oder Polypeptide vor dem Binden an den Träger nicht gereinigt werden.

8. Verfahren nach mindestens einem der Ansprüche 1 bis 7, **dadurch gekennzeichnet, dass** es ferner die Schritte zur Normierung der Menge an synthetisiertem Protein vor dem Binden an einen Träger umfasst, wobei die Normierungsschritte aus folgendem bestehen:

a) Markieren jedes synthetisierten Proteins, vorzugsweise durch Zuführen einer radioaktiven Aminosäure in das zellfreie System,

b) Quantifizieren jedes markierten Proteins, und

c) Vorsehen einer äquivalenten Menge jedes synthetisierten Proteins vor dem Binden an einen Träger zur Normierung.

9. Verfahren nach mindestens einem der Ansprüche 1 bis 8, wobei mindestens zwei verschiedene Sonden verwendet werden, wobei die Sonden mit IR-Farbstoffen von verschiedener Wellenlänge markiert sind.

10. Verfahren nach mindestens einem der Ansprüche 1 bis 9, wobei die Nah-Infrarot-Fluoreszenzfarbstoffe eine Wellenlänge über 680 nm aufweisen.

11. Verfahren zur Herstellung eines Protein-Arrays, umfassend Proteinziele, vorzugsweise einschließlich eines Proteins oder Polypeptids, das *in vivo* nicht-herstellbar ist, welches bei Expression *in vivo* in Wirtszellen toxisch für die Wirtszellen ist oder nicht gereinigt werden kann oder sich nicht korrekt faltet, wobei das Verfahren folgendes umfasst:

a) paralleles Synthetisieren von Proteinzielen unter Anwendung eines zellfreien Proteinsyntheseverfahrens mit einem Überschuss der α-Untereinheit von RNA-Polymerase entsprechend anderen Untereinheiten derselben;

b) Gewinnen der synthetisierten Proteine oder Polypeptide; und

c) Herstellen eines Protein-Arrays durch Binden von synthetisierten Proteinen oder Polypeptiden an einen Träger an definierten Positionen.

12. Verfahren nach Anspruch 11, wobei das zellfreie Proteinsyntheseverfahren die Verwendung eines bakteriellen zellfreien Extraktes, bevorzugt eines zellfreien *E.coli*-Extraktes, umfasst.

13. Verfahren nach Anspruch 11 oder 12, wobei das zellfreie Proteinsyntheseverfahren die Verwendung einer PCR-erzeugten DNA-Matrize umfasst, wobei die PCR-erzeugte DNA-Matrize einen das Protein oder Polypeptid codierenden offenen Leserahmen und eine zusätzliche DNA-Sequenz, welche mindestens 3 bp lang, vorzugsweise länger als 100 bp und weiter bevorzugt länger als 200 bp ist, die unmittelbar stromabwärts des Stop-Codons des das Protein oder Polypeptid codierenden offenen Leserahmens lokalisiert ist, umfasst.

14. Verfahren nach mindestens einem der Ansprüche 11 bis 13, wobei die DNA-Matrize einen starken Promotor mit mindestens einem UP-Element umfasst, der stromaufwärts des ORF lokalisiert ist.

15. Verfahren gemäß den Ansprüchen 12 bis 14, wobei eine gereinigte thermostabile RNA-Polymerase, vorzugsweise aus *T. thermophilus,* in den bakteriellen zellfreien Extrakt zugesetzt wird.

16. Verfahren nach mindestens einem der Ansprüche 11 bis 15, wobei die synthetisierten Proteine oder Polypeptide vor dem Binden an den Träger nicht gereinigt werden.

17. Verwendung von Zielproteinen für die Herstellung von Protein-Arrays, wobei die Zielproteine auf dem Array als ein Rohextrakt abgeschieden werden, ohne Reinigung nach ihrer Synthese, der erhalten wird durch ein zellfreies Proteinsynthesesystem mit einem Überschuss der α-Untereinheit von RNA-Polymerase im Vergleich zu anderen Untereinheiten derselben.

18. Verwendung nach Anspruch 17, wobei das zellfreie Proteinsynthesesystem einen bakteriellen zellfreien Extrakt, vorzugsweise zellfreien *E.coli*-Extrakt, umfasst.

19. Verfahren nach Anspruch 18, wobei die in dem Rohextrakt enthaltenen Proteine oder Polypeptide synthetisiert werden von einer PCR-erzeugten DNA-Matrize, wobei die PCR-erzeugte DNA-Matrize den das Protein oder Polypeptid codierenden offenen Leserahmen und eine zusätzliche Sequenz, welche mindestens 3 bp lang, vorzugsweise länger als 100 bp und weiter bevorzugt länger als 200 bp ist, die unmittelbar stromabwärts des Stop-Codons des das Protein oder Polypeptid codierenden Gens lokalisiert ist, umfasst.

20. Verwendung nach mindestens einem der Ansprüche 17 bis 19, wobei die DNA-Matrize DNA-Matrize einen starken Promotor mit mindestens einem UP-Element umfasst, der stromaufwärts des ORF lokalisiert ist.

21. Verfahren gemäß den Ansprüchen 17 bis 20, wobei eine gereinigte thermostabile RNA-Polymerase, vorzugsweise aus *T. thermophilus,* in den bakteriellen zellfreien Extrakt zugesetzt wird.

**22.** Verfahren gemäß den Ansprüchen 17 bis 21, wobei mindestens eines von dem Protein oder Polypeptid bei Expression *in vivo* in Wirtszellen toxisch für die Wirtszellen ist oder nicht gereinigt werden kann oder sich nicht korrekt faltet.

**Revendications**

**1.** Procédé de détection d'interactions intermoléculaires entre une/des sonde(s) et des cibles protéiques, lesdites cibles protéiques incluant de préférence au moins une protéine ou un polypeptide non productible *in vivo* qui, exprimé(e) *in vivo* dans des cellules hôtes, est toxique pour lesdites cellules hôtes, ou ne peut pas être purifié(e), ou ne se replie pas correctement, ledit procédé combinant les étapes de :

a) fourniture de cibles protéiques par synthèse *in vitro* en série de celles-ci, en utilisant un procédé de synthèse protéique acellulaire avec un excès de la sous-unité α d'ARN polymérase en comparaison des autres sous-unités de celle-ci ;
b) liaison, à un support à des positions définies, desdites cibles protéiques pour préparer un réseau de protéines ;
c) fourniture d'une/de sonde(s) marquée(s) avec un colorant fluorescent dans le proche infrarouge ;
d) mise en contact dudit réseau de protéines avec la/les sonde(s) marquée(s) dans un milieu approprié pour permettre une liaison spécifique ; et
e) détection sur le réseau de protéines de la/des cible(s) protéique(s) à laquelle/auxquelles la/les sonde(s) marquée(s) se lie(nt) spécifiquement en utilisant un dispositif d'imagerie infrarouge.

**2.** Procédé selon la revendication 1, dans lequel ledit procédé de synthèse protéique acellulaire comprend l'utilisation d'un extrait acellulaire, de préférence un extrait acellulaire bactérien, de préférence un extrait acellulaire de *E. coli.*

**3.** Procédé selon la revendication 1 ou 2, dans lequel le système de synthèse protéique acellulaire comprend l'utilisation d'une matrice d'ADN produite par PCR, ladite matrice d'ADN produite par PCR comportant un cadre de lecture ouvert codant pour ladite protéine ou ledit polypeptide, et une séquence d'ADN supplémentaire, longue d'au moins 3 bp, de préférence plus longue que 100 bp, et plus préférentiellement plus longue que 200 bp, située immédiatement en aval du codon de terminaison du cadre de lecture ouvert codant pour ladite protéine ou ledit polypeptide.

**4.** Procédé selon l'une quelconque des revendications 1 à 3, dans lequel ladite matrice d'ADN comprend un promoteur fort avec au moins un élément UP situé en amont de l'ORF.

**5.** Procédé selon les revendications 2 à 4, dans lequel ladite ARN polymérase est une polymérase thermostable purifiée, de préférence de *T. thermophilus.*

**6.** Procédé selon l'une quelconque des revendications 1 à 5, dans lequel ladite molécule sonde est un acide nucléique, un polypeptide ou une protéine marqué(e) avec un colorant fluorescent dans le proche infrarouge.

**7.** Procédé selon l'une quelconque des revendications 1 à 6, dans lequel lesdites protéines ou lesdits polypeptides synthétisé(e)s ne sont pas purifié(e)s avant liaison au support.

**8.** Procédé selon l'une quelconque des revendications 1 à 7, **caractérisé en ce qu'**il comprend en outre des étapes pour normalisation de la quantité de protéine synthétisée avant liaison au support, lesdites étapes de normalisation consistant à :

a) marquer chaque protéine synthétisée, de préférence en fournissant un acide aminé radioactif dans le système acellulaire,
b) quantifier chaque protéine marquée, et
c) fournir une quantité équivalente de chaque protéine synthétisée avant liaison au support pour normalisation.

**9.** Procédé selon l'une quelconque des revendications 1 à 8, dans lequel au moins deux sondes différentes sont utilisées, lesdites sondes étant marquées avec des colorants IR ou de longueur d'onde différente.

**10.** Procédé selon l'une quelconque des revendications 1 à 9, dans lequel lesdits colorants fluorescents dans le proche infrarouge possèdent une longueur d'onde supérieure à 680 nm.

**11.** Procédé de préparation d'un réseau de protéines comprenant des cibles protéiques, incluant de préférence une protéine ou un polypeptide non productible *in vivo* qui, exprimé(e) *in vivo* dans des cellules hôtes, est toxique pour lesdites cellules hôtes, ou ne peut pas être purifié(e), ou ne se replie pas correctement, ledit procédé comprenant :

a) la synthèse en parallèle de cibles protéiques en utilisant un procédé de synthèse protéique acellulaire avec un excès de la sous-unité α d'ARN polymérase par rapport aux autres sous-unités de celle-ci ;
b) la récupération des protéines ou des polypeptides synthétisé(e)s ; et
c) la préparation d'un réseau de protéines en liant les protéines ou les polypeptides synthétisé(e)s à un support à des positions définies.

**12.** Procédé selon la revendication 11, dans lequel ledit procédé de synthèse protéique acellulaire comprend l'utilisation d'un extrait acellulaire bactérien, de préférence un extrait acellulaire de *E. coli.*

**13.** Procédé selon la revendication 11 ou 12, dans lequel ledit procédé de synthèse protéique acellulaire comprend l'utilisation d'une matrice d'ADN produite par PCR, ladite matrice d'ADN produite par PCR comprenant le cadre de lecture ouvert codant pour ladite protéine ou ledit polypeptide, et une séquence d'ADN supplémentaire, longue d'au moins 3 bp, de préférence plus longue que 100 bp, et plus préférentiellement plus longue que 200 bp, située immédiatement en aval du codon de terminaison dudit cadre de lecture ouvert codant pour ladite protéine ou ledit polypeptide.

**14.** Procédé selon l'une quelconque des revendications 11 à 13, dans lequel ladite matrice d'ADN comprend un promoteur fort avec au moins un élément UP situé en amont de l'ORF.

**15.** Procédé selon les revendications 12 à 14, dans lequel une ARN polymérase thermostable purifiée, de préférence de *T. thermophilus,* est ajoutée dans l'extrait acellulaire bactérien.

**16.** Procédé selon l'une quelconque des revendications 11 à 15, dans lequel lesdites protéines ou lesdits polypeptides synthétisé(e)s ne sont pas purifié(e)s avant liaison au support.

**17.** Utilisation de protéines cibles pour la préparation de réseaux de protéines, dans laquelle lesdites protéines cibles sont déposées sur le réseau sous la forme d'un extrait brut, sans purification, après leur synthèse obtenue par un système de synthèse protéique acellulaire avec un excès de la sous-unité α d'ARN polymérase en comparaison des autres sous-unités de celle-ci.

**18.** Utilisation selon la revendication 17, dans laquelle ledit système de synthèse protéique acellulaire comprend un extrait acellulaire bactérien, de préférence un extrait acellulaire de *E. coli.*

**19.** Utilisation selon la revendication 18, dans laquelle lesdites protéines ou lesdits polypeptides contenu(e)s dans l'extrait brut sont synthétisé(e)s à partir d'une matrice d'ADN produite par PCR, ladite matrice d'ADN produite par PCR comprenant le cadre de lecture ouvert codant pour ladite protéine ou ledit polypeptide, et une séquence supplémentaire, longue d'au moins 3 bp, de préférence plus longue que 100 bp, et plus préférentiellement plus longue que 200 bp, située immédiatement en aval du codon de terminaison du gène codant pour ladite protéine ou ledit polypeptide.

**20.** Utilisation selon l'une quelconque des revendications 17 à 19, dans laquelle ladite matrice d'ADN comprend un promoteur fort avec au moins un élément UP situé en amont de l'ORF.

**21.** Utilisation selon les revendications 17 à 20, dans laquelle une ARN polymérase thermostable purifiée, de préférence de *T. thermophilus,* est ajoutée dans l'extrait acellulaire bactérien.

**22.** Utilisation selon les revendications 17 à 21, dans laquelle au moins un(e) des protéines ou des polypeptides, exprimé (e)s *in vivo* dans des cellules hôtes, est toxique pour lesdites cellules hôtes, ou ne peut pas être purifié(e), ou ne se replie pas correctement.

Figure 1

Figure 2

Figure 3

Figure 4

Figure 5a

EP 1 412 758 B1

**(b)**

*B. stearothermophilus* **ArgR**

**Figure 5b**

**(c)**

**Chimeric ArgR-EbE**

Figure 5c

EP 1 412 758 B1

Figure 5d

**Binding *E. coli* CRP to *B. stearothermophilus* PargCo**

Figure 6

Figure 7

**Figure 8**

ArgR *T. neapolitana*

Tm0808

Tm1856

Tm0439

**Figure 9**

**a**

**GntTM**

0766 0275 0439 CRP α Arg_{Tn} ArgR_{Ec}

**b**

**LacTm**

1856 1218 0299 0949 1200

**c**

**·XylTm**

0393 0808 0032 1224 0110 0411

**Figure 10**

Figure 11

Figure 12

Figure 13

**a** ArgR *E. coli*
**b** ArgR *B. stearothermophilus*
**c** ArgR *T. neapolitana*

Binding to IRD-800 59-bp DNA
**d** **e** **f**

Binding to IRD-800 39-bp DNA
**g** **h** **i**

Binding to Cy5.5 αRNP *E. coli*
**j** **k** **l**

Fig. 14

Fig. 15